(19) Europäisches Patentamt / European Patent Office / Office européen des brevets

(11) **EP 4 586 881 B1**

(12) **EUROPEAN PATENT SPECIFICATION**

(45) Date of publication and mention of the grant of the patent:
**14.01.2026 Bulletin 2026/03**

(51) International Patent Classification (IPC):
**A61B 3/032** (2006.01)    **A61B 3/036** (2006.01)
**A61B 3/15** (2006.01)    **A61B 3/103** (2006.01)
**A61B 3/107** (2006.01)

(21) Application number: **23809228.2**

(52) Cooperative Patent Classification (CPC):
**A61B 3/036; A61B 3/032;** A61B 3/1035;
A61B 3/107; A61B 3/152

(22) Date of filing: **20.11.2023**

(86) International application number:
**PCT/EP2023/082429**

(87) International publication number:
**WO 2024/110403 (30.05.2024 Gazette 2024/22)**

(54) **COMPUTER-IMPLEMENTED METHOD AND DEVICES FOR DETERMINING AT LEAST ONE ASTIGMATISM PARAMETER OF AT LEAST ONE EYE OF A PERSON**

COMPUTERIMPLEMENTIERTES VERFAHREN UND VORRICHTUNGEN ZUR BESTIMMUNG MINDESTENS EINES ASTIGMATISMUSPARAMETERS MINDESTENS EINES AUGES EINER PERSON

PROCÉDÉ ET DISPOSITIFS MIS EN UVRE PAR ORDINATEUR POUR DÉTERMINER AU MOINS UN PARAMÈTRE D'ASTIGMATISME D'AU MOINS UN OEIL D'UNE PERSONNE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **21.11.2022 EP 22208605**

(43) Date of publication of application:
**23.07.2025 Bulletin 2025/30**

(73) Proprietor: **Carl Zeiss Vision International GmbH 73430 Aalen (DE)**

(72) Inventors:
• **DOBBELSTEIN, David**
  **73447 Oberkochen (DE)**
• **STOPPE, Lars**
  **73447 Oberkochen (DE)**
• **MEINHARDT, Luca-Maxim**
  **73447 Oberkochen (DE)**
• **WALD, Matthias**
  **73447 Oberkochen (DE)**
• **LEUBE, Alexander**
  **73430 Aalen (DE)**

(74) Representative: **Altmann Stößel Dick Patentanwälte PartG mbB Theodor-Heuss-Anlage 2 68165 Mannheim (DE)**

(56) References cited:
**WO-A1-2022/090376    KR-B1- 101 784 599
US-A1- 2013 235 346    US-A1- 2016 106 581
US-A1- 2019 298 168**

**Description**

The present invention relates to a computer-implemented method for determining at least one astigmatism parameter of at least one eye of a person, a computer program, a remote device for generating outcome data for determining at least one astigmatism parameter of at least one eye of a person, a determining device for determining at least one astigmatism parameter of an eye of a person, a method for producing a geometrical model of at least one spectacle lens for manufacturing of the at least one spectacle lens, a method for producing at least one spectacle lens, and a use of an external lens.

Related art

Typically, astigmatism parameters of a person are determined by using stationary keratometer, also known as ophthalmometer. These devices are diagnostic instruments that may be used by an expert for measuring the curvature of the anterior surface of the cornea, particularly for assessing the extent and axis of astigmatism.

US 9 833 140 B2 discloses portable corneal topographers and portable corneal topographer modules. In one embodiment, a corneal topographer module comprises a housing having a first aperture and a second aperture formed there through; and a plurality of optical components disposed within the housing. The plurality of optical components are arranged to direct light generated by a light source through the first aperture to the cornea via a first optical channel when a cornea of a patient's eye is located adjacent to the first aperture; and direct reflected light from the cornea into the housing through the first aperture and to a light detector of a mobile device via a second optical channel when the corneal topographer module is mechanically coupled to the mobile device.

US 2020/0237210 A1 discloses demonstrative apparatuses, systems and/or methods of determining one or more parameters of a refractive error of a tested eye. For example, a computing device may be configured to process depth mapping information to identify depth information of a tested eye; and to determine one or more parameters of a refractive error of the tested eye based on the depth information of the tested eye.

US 2017/0172406 A1 discloses in illustrative implementations that a human user mechanically moves one or more moveable parts in a handheld controller, and thereby optically controls a mobile computing device. In illustrative implementations, the optical control is implemented as follows: A camera onboard the mobile computing device captures images. The images show the motion of the moveable parts in the handheld controller. A camera onboard the mobile computing device analyzes these images to detect the motion, maps the motion to a control signal, and outputs a control signal that controls a feature or operation of the mobile computing device.

US2017/0164827 A1 discloses mobile computer devices and systems for refraction determination of an eye, for example for objective refraction determination and/or subjective refraction determination. Here, a display of the mobile computer device can be driven to display an image for refraction determination.

US 2014/0268060 A1 discloses generally a system and method for determining the refractive error of a patient, more particularly determining the patient's refractive error by using a computerized screen, and providing the patient with a prescription for the patient's preferred type of corrective lenses. The system and method do not require the trip or expense of a doctor visit, and are optimized for convenience and cost effectiveness. In a general embodiment, the present disclosure provides a method for determining a corrective lenses prescription of a patient. The method includes, separately, for each eye of the patient, determining the astigmatism prescription of the patient via a computerized screen, and determining the power of the corrective lenses prescription of the patient via the computerized screen.

DE 10 2015 100 147 A1 discloses mobile computer devices, mounts, and corresponding computer program products, which may be used to determine a characteristic of the eye, for example, for diagnostic purposes.

KR 101 784 599 B1 discloses an eye measurement system including a portable terminal that displays an eye state detection pattern, a lens unit that projects the eye state detection pattern onto the eye, a light source that irradiates light to the eye and an eye state reflected from the eye.

WO 2022/090376 Al discloses a method, computer program and a device for determining at least one refractive error of at least one eye of a person using at least one mobile communication device. Herein, the method comprising the following steps: a) displaying at least one illumination pattern to at least one eye of a person; b) capturing at least one image picturing at least one reflection of the at least one illumination pattern illuminating at least one portion of the at least one eye of the person; and c) determining a value for at least one refractive error of the at least one eye of the person by processing the at least one image, wherein the at least one mobile communication device comprises a screen which is configured to perform step a) and at least one camera which is configured to perform step b).

Problem to be solved

It is therefore an objective of the present invention, particularly in view of DE 10 2015 100 147 A1, to provide a computer-implemented method for determining at least one astigmatism parameter of at least one eye of a person, a computer program, a field device for generating input data for determining at least one astigmatism parameter of at least one eye of a person, a remote device for generating outcome data for determining at least one astigmatism parameter of at least one eye of a person, a data carrier signal, a determining device for determining at least one astigmatism parameter of an eye of a person, a method for producing a geometrical model of at least one spectacle lens for manufacturing of the at least one spectacle lens, a method for producing at least one spectacle lens and a use of an external lens, which at least partially overcome the above-mentioned problems of the state of the art.

It is a particular objective of the present invention to provide a fast, easy, versatile, reliable, easy accessible and accurate approach for determining at least one astigmatism parameter of at least one eye of a person.

## Summary of the invention

This problem is solved by a computer-implemented method for determining at least one astigmatism parameter of at least one eye of a person according to claim 1, a computer program according to claim 9, a field device not forming part of the claimed invention for generating input data for determining at least one astigmatism parameter of at least one eye of a person, a remote device for generating outcome data for determining at least one astigmatism parameter of at least one eye of a person according to claim 10, a data carrier signal not forming part of the claimed invention, a determining device for determining at least one astigmatism parameter of an eye of a person according to claim 11, a method for producing a geometrical model of at least one spectacle lens for manufacturing of the at least one spectacle lens according to claim 12, a method for producing at least one spectacle lens according to claim 13, and a use of an external lens according to claim 14. Preferred embodiments are listed in the dependent claims.

In a first aspect, the present invention relates to a computer-implemented method for determining at least one astigmatism parameter of at least one eye of a person according to claim 1.

As generally used, the term "computer-implemented method" refers to a method which involves at least one apparatus, specifically a computer, or a plurality of apparatuses, particularly connected via a computer network. The plurality of apparatuses may be connected, particularly for transmitting data, via a network by using at least one connection interface at any one of the apparatuses of the plurality of the apparatuses. The computer-implemented method may be implemented as at least one computer program that may be provided on a storage medium carrying the computer program, whereby at least one of the steps of the computer-implemented method, specifically at least one of steps a) or b), are performed by using the at least one computer program. Preferably, any one of the steps a) and b) are performed using the at least one computer program. Alternatively, the at least one computer program may be accessible by an apparatus which may be adapted for performing the method via a network, such as via an in-house network, via internet, or via a cloud. With particular regard to the present invention, the present method can, thus, be performed on a programmable apparatus, which is configured for this purpose, such as by providing a computer program, which is configured for such a purpose.

As generally used, the term "determining" or any grammatical variation thereof refers to a process configured for generating at least one representative result. The representative result may be determined in a process, wherein at least one step of the process may be selected from at least one of: a measurement step; an evaluation step; or a displaying step. For determining the representative result at least one measurement may be performed. Data generated in this measurement may be evaluated. The representative result may be data retrieved in the evaluation process. The representative result may be displayed. With particular regard to the present invention, the representative result comprises outcome data comprising at least one astigmatism parameter of the at least one eye of the person. The outcome data may be displayed on at least one screen.

As generally used, the term "astigmatism" refers to an aspheric refractive defect in which an eye has different refractions in at least two sections, particularly due to a topography of the at least one eye of the person. The eye may be considered to suffer from astigmatism in case its surface is not rotationally symmetric. As generally used, the term "astigmatism parameter" refers to a value and/or measure of the aspheric defect of the eye.

The method may be performed for exactly one eye of the person simultaneously. The method may be repeated for a further eye of the person. The method may further be implemented for being performed for at least one eye of the person or both eyes of the person or any eye of the person simultaneously.

The method for determining at least one astigmatism parameter of at least one eye of a person comprises the following steps, which may be performed in the given order. A different order, however, may also be feasible. Further, two or more of the method steps may be performed simultaneously. Thereby the method steps may at least partly overlap in time. Further, the method steps may be performed once or repeatedly. Thus, one or more or even all of the method steps may be performed once or repeatedly. The method may comprise additional method steps, which are not listed herein.

The method is defined in claim 1 and comprises, inter alia, the following steps:

a) generating input data configured to comprise

- at least one known feature configured for determining the at least one astigmatism parameter of the at least one eye of the person;
- at least one image of at least one reflected feature in the eye of the person, wherein the at least one image of the at least one reflected feature is generated, in a step i), by displaying the at least one known feature on a measurement device in a manner that the at least one known feature is reflected by the eye of the person; wherein the at least one image of the at least one reflected feature in the eye of the person is recorded, in a step ii), by using the measurement device;
- at least one position of the at least one eye of the person relative to the measurement device; wherein the at least one position of the at least one eye of the person is determined, in a step iii), by using the measurement device;

b) generating outcome data, particularly by using a processing device, configured to comprise

- at least one astigmatism parameter of the eye of the person; wherein the at least one astigmatism parameter of the eye of the person is determined, in a step iv), by comparing the at least one known feature to the at least one reflected feature comprised in the at least one recorded image of the at least one reflected feature in the eye of the person, wherein the position of the at least one eye of the person is considered when the at least one astigmatism parameter of the eye of the person is determined.

According to step a), input data is generated. As used herein, the term "generating" or any grammatical deviation thereof refers to a process of producing a set or a sequence of data, particularly by taking an initial set of data, specifically measurement data, and applying a mathematical or logical process to it. The term does not necessarily imply that a measurement process occurs. For generating input data, it may be sufficient to provide and/or collect at least one piece of input data that is comprised by the input data. Thereby, the process may make use of an algorithm and/or a machine learning model. The produced set of data may be the input data. As used herein, the term "data" refers to a set or sequence of information provided in a machine readable fashion. Any item comprised by the data may be comprised as piece of information and/or information and/or as a digital representation. As used herein, the term "input data" refers to a set or sequence of data that comprises information that are at least partly evaluated by a computer-implemented method, particularly the input data may be required for starting at least one step of the method, specifically the computer-implemented method for determining at least one astigmatism parameter of an eye of a person. The input data may be required for generating the outcome data.

The input data according to step a) is configured to comprise

- at least one known feature configured for determining the at least one astigmatism parameter of the at least one eye of the person;
- at least one image of at least one reflected feature in the eye of the person, wherein the at least one image of the at least one reflected feature is generated, in a step i), by displaying the at least one known feature on a measurement device in a manner that the at least one known feature is reflected by the eye of the person; wherein the at least one image of the at least one reflected feature in the eye of the person is recorded, in a step ii), by using the measurement device;
- at least one position of the at least one eye of the person relative to the measurement device; wherein the at least one position of the at least one eye of the person is determined, in a step iii), by using the measurement device.

As used herein, the term "measurement device" refers to an apparatus and/or a device, such as a field device and/or a determining device as described elsewhere herein, that is being used for performing the computer-implemented method for determining at least one astigmatism parameter of at least one eye of a person. The measurement device may comprise a screen and/or an image capturing unit. Further, the measurement device may comprise a distance measurement device.

As used herein, the term "feature" refers to a recognizable characteristic that distinguishes an item from further items. The respective item may thus be recognized. A feature may differ from at least one further item, for example in color, grey scale, image gradients and/or intensity. The feature may be configured for determining at least one aspheric refractive defect in the at least one eye of the person. The feature may be configured for determining a shape of a surface of the eye of the person.

The term "known feature" refers to a feature that is available and/or given for determining the at least one astigmatism parameter of the at least one eye of the person. The known feature may be configured for determining the at least one astigmatism parameter of the at least one eye of the person. Particularly, a shape or at least one part of a shape, specifically an expansion, of the known feature may be available and/or given. Thereby, the at least one astigmatism parameter may be determined by considering the known feature, particularly the shape or at least one part of the shape, specifically the expansion.

The term "reflected feature" refers to a feature that is reflected on the eye of the person. Thereby, the reflected feature is visible in the eye of the person. The reflected feature, particularly a shape or at least one part of a shape, specifically an expansion, may be influenced by at least one refractive effect, such as the aspheric refractive defect, of the at least one eye of the person. Alternatively or in addition, the reflected feature, particularly the shape or the at least one part of the shape, specifically the expansion, may be influenced by the shape of the surface of the eye of the person. At least one of these influences may cause a specific deformation of the reflected pattern relative to the known pattern being displayed on the at least one measurement device, particularly the screen. Thereby, the at least one astigmatism parameter may be determined by considering the reflected feature, particularly the shape or at least one part of the shape, specifically the expansion. Alternatively or in addition, the at least one astigmatism parameter may be determined by comparing the known feature to the reflected feature, particularly the shape or at least one part of the shape, specifically the expansion, of both features.

The term "image" refers to data configured to comprise information about the at least one reflected feature, particularly information about the shape or the at least one part of the shape, more specifically the expansion. The image may be a photograph, or an image frame from a video sequence. Further types of images are possible.

Again, the measurement device may comprise a screen configured for displaying the at least one known feature and/or an image capturing unit configured for recording an image of the at least one reflected feature on the eye of the person. As generally used, the term "displaying" or any grammatical deviation thereof refers to a presentation of at least one of an image, an item, a text, or a video, particularly at the at least one known feature, specifically on the screen. As generally used, the term "recording" or any grammatical variation thereof refers to a process of generating data, particularly comprised by the input data. The data may be generated in a measurement process, specifically by recording the image.

As used herein, the term "position of the at least one eye" refers to a location of the at least one eye of the user in relation to the measurement device. More specifically the position of the at least one eye of the user may be determined relative to the screen and/or the known feature, particularly a center of the known feature. Alternatively or in addition, the at least one known feature may be determined relative to at least one distance measurement device comprised by the measurement device.

For determining the position relative to the at least one eye of the person, the measurement device may comprise the image capturing unit configured for determining the position of the at least one eye of the person, particularly in a plane, and/or the measurement device may comprise a distance measurement device configured for determining a distance of the at least one eye of the person to the measurement device, particularly the distance of the eye in the plane, wherein the plane is orthogonal to the distance.

The arrangement of at least one of: the screen, the image capturing unit, or the distance measurement device may be known, specifically for a specific type and/or model of the at least one measurement device. The respective arrangement may be given in calibration data, specifically by reading out a database configured to comprise the information about the respective arrangement. The calibration data may be acquired in a calibration process. The respective arrangement may be considered for determining the at least one astigmatism parameter, particularly for determining the position of the at least one eye of the user relative to the screen and/or to the displayed known feature, particularly a center of the displayed known feature. Thus, the input data may comprise information on the type and/or model of the at least one measurement device. During the determination of the at least one astigmatism parameter, the database configured to comprise the information about the respective arrangement may be read out for considering the respective arrangement.

Any information recorded by the image capturing unit and/or the distance measurement device, particularly information about spatial location, such as the position of the eye of the person, may be given in a respective coordinate system. The respective coordinate system may be transferred to and/or be given in a world coordinate system. For transferring both respective coordinate system into one another and/or for transferring at least one respective coordinate system or both respective coordinate systems into the world coordinate system a transformation rule, exemplarily given in a transfer matrix, may be applied, such a transformation rule retrieved from a calibration process. The input data may comprise the relevant transformation rule

According to step b), outcome data is generated. As used herein, the term "outcome data" refers to a set or sequence of data that comprises information determined in at least one step of a computer-implemented method, specifically the computer-implemented method for determining at least one astigmatism parameter of the eye of a person. The outcome data may comprise the representative result, particularly determined by the computer-implemented method for determining at least one astigmatism parameter of the eye of the person. For generation the outcome data, input data may be considered and/or evaluated.

The outcome data according to step b) is configured to comprise

-    at least one astigmatism parameter of the eye of the person; wherein the at least one astigmatism parameter of the eye of the person is determined, in a step iv), by comparing the at least one known feature to the at least one reflected feature comprised in the at least one recorded image of the at least one reflected feature in the eye of the person, wherein the position of the at least one eye of the person is considered when the at least one astigmatism parameter of the eye of the person is determined.

As used herein, the term "comparing" refers to a process of analyzing a potential deformation between the reflected feature and the displayed known feature, particularly by determining a deviation between the reflected feature and the displayed known feature. The deformation may be caused by an optical defect of the at least one eye of the person, such as the aspheric refractive defect. The refractive defect may be related to the shape, particularly the topography, of the at least one eye of the person. By analyzing the deviation between the reflected feature and the displayed known feature, the at least one astigmatism parameter may be determined.

As used herein, the term "reflected feature comprised in the at least one recorded image of the at least one reflected feature in the eye of the person" refers to information about the reflected feature, specifically the shape or the at least one part of the shape, more specifically the expansion, being included in the at least one recorded image.

As generally used, the term "considering" or any grammatical variation thereof, specifically "considered", refers to a process of taking at least one factor into account, particularly for and/or when determining a representative result, such as the astigmatism parameter. The representative result may depend on the at least one factor in a manner that the result, specifically the astigmatism parameter, is different for at least two different factors. The position of the eye of the person relative to the measurement device may influence the determined at least one astigmatism parameter.

The outcome data may be presented to the at least one person, particularly by displaying the outcome data, specifically the at least one astigmatism parameter, on the at least one screen or on a further screen or monitor.

For further details concerning the method, a reference may be made to any aspect and/or any definition according to the present invention as disclosed elsewhere herein.

The at least one known feature may be displayed, particularly in step i), on a screen comprised by the measurement device for projecting the at least one feature on the at least one eye of the person. As generally used, the term "screen" refers to an electronic visual display device configured for the presentation of at least one of an image, an item, text, and/or a video. Alternatively or in addition, the screen may be configured for presenting the at least one known feature. The screen may be comprised by or be part of the measurement device.

The image of the at least one reflected feature on the eye of the person may be recorded, particularly in step ii), by using an image capturing unit comprised by the measurement device. As generally used, the term "image capturing unit" refers to a device configured for recording at least one image comprising information about the at least one reflected feature. The image capturing unit may be at least one camera of the measurement device.

Determining the at least one position of the eye of the person, particularly in step iii), may comprise determining a distance of the at least one eye of the person to the measurement device, particularly by using the image capturing unit and/or a distance measurement device.

As generally used, the term "distance" refers to a length of the shortest connection between two points. The first point may be the at least one eye of the person. The second point may be a center of an abstract line connecting the image capturing unit, particularly the center of the image capturing unit, and the displayed known feature, particularly a center of the displayed known feature. Determining the at least one position of the eye of the person, particularly in step iii), may comprise determining a position of the eye of the person in a plane, particularly wherein the plane is perpendicular and/or orthogonal to the distance, particularly by using the image capturing unit and/or an distance measurement device. As generally used, the term "plane" refers to a two-dimensional surface, particularly defined by two orthogonal vectors. By considering the distance and the plane a three-dimensional position of the at least one eye of the person may be determined and/or known, specifically in relation to and/or relative to the at least one measurement device.

The at least one position of the at least one eye of the person in relation to and/or relative to the measurement device may be determined directly before, directly after and/or when the image of the at least one reflected feature on the eye of the person may be recorded, particularly in step ii). Thereby, the position considered during the determination of the at least one astigmatism parameter is the position of the at least one eye of the person in relation to the measurement device at the time the at least one image of at least one reflected feature in the eye of the person is recorded. As used herein, the term "directly" refers to the position being determined, particularly by recording image recording data, before or after the image of the at least one reflected feature on the eye of the person may be recorded, wherein a time range between the determination of the at least one position and the recording of the at least one image is below a threshold. This threshold may, typically, be 1 s; 500 ms; 100 ms; or 10 ms. As used herein, the term "when" refers to the image being recorded at the same time as the position of the at least one eye of the person in relation to and/or in relative to the measurement device may be determined.

Determining the distance of the at least one eye of the person to the measurement device may comprise at least one of the following steps:

- recording at least one depth field map by using the measurement device , particularly a distance measurement device comprised by the measurement device;

- determining the location of the at least one eye of the person in the at least one depth field map, particularly by at least one of:

    ◦ using at least one image analysis procedure on the recorded at least one depth field map; or
    ◦ comparing the recorded at least one depth field map to at least one recorded image showing the at least one eye of the person at the time the at least one depth field map is recorded, wherein the position of the at least one eye of the person in the at least one recorded image showing the at least one eye of the person is determined by using at least on image analysis procedure;

- determining the distance of the at least one eye of the person from the recorded at least one depth field map by considering the determined location of the at least one eye of the person in the at least one depth field map.

As generally used, the term "depth field map" refers to a monochromatic two-dimensional image comprising three dimensional information. A portion of the three dimensional information, such as two dimension information, may derived from considering a property configured to comprise the two dimensional information, such as a location of a pixel on the two dimension image. A further portion of the three dimensional information, such as a one dimensional information, may be derived from considering a further property configured to comprise the two dimensional information, such as a gray scale, of the pixel. Exemplarily, a pixel that is far away from the distance measurement device is shown in black and a pixel that is close to the distance measurement device is represented in white.

As generally used, the term "distance measurement device" refers to a device configured for recording information about a distance. The information may be a value related to or may be a value of the distance. The distance may be the distance between the eye of the person and the measurement device, particularly the distance measurement device comprised by the measurement device. The arrangement between the screen and/or the image capturing unit may be known from calibration data, specifically by considering a database. The distance measurement device may be configured for providing and/or recoding, such as in a measurement process, the depth field map.

As generally used, the term "image analysis procedure" refers to a method in which the depth field may be analyzed for determining the location of the at least one eye of the person in the at least one depth field map. This analysis procedure may comprise using at least one of: a machine learning model, an image analysis procedure, such as knowledge based, feature based, appearance based, and/or template matching face detection methods. These methods may be considered as plausibility checks of the depth field map. As generally used, the term "machine learning model" refers to a trainable computer-implemented architecture, particularly a trainable statistical model, that applies artificial intelligence to automatically determine a representative result, particularly the location of the at least one eye of the person. As generally used, the term "training" refers to a process of determining adjustable parameters of the machine learning model using a training data for generating a trained machine learning model. The training may comprise at least one optimization or tuning process, wherein a best parameter combination is determined. The training is carried out to improve the capability of the machine learning model to determine the representative result, particularly a location of the at least one eye of the person, by analyzing at least a portion of the training data.

Determining the distance of the at least one eye of the person to the measurement device may comprise performing an image analysis procedure of the at least one recorded image showing the at least one eye of the person, particularly by at least one of the following steps:

- considering at least one target having a known target size in at least one recorded image showing the at least one eye of the person; particularly wherein the at least one target is selected from at least one of:

    ◦ a credit card; or
    ◦ a property of the at least one eye of the person, specifically an iris of the at least one eye of the person;

- determining the size of the at least one target in the at least one recorded image showing the at least one eye of the person, particularly by using at least one image analysis procedure;
- determining the distance by considering the size of the at least one target, particularly by performing a triangulation procedure.

For measuring the at least one distance, the at least one distance measurement device, particularly the at least one image capturing unit, more particularly the respective camera, may be calibrated by using standard calibration methods known to the person skilled in the art.

As generally used, the term "triangulation procedure" refers to method in which a triangle is assumed having known parameters and wherein missing parameters of the triangle are determined by considering the missing parameters. Exemplarily, by using trigonometry and considering the known angles of the triangle and a known length of one side, the further distances in the triangle may be calculated.

The size of the at least one target at a predetermined distance may be determined by considering at least one recorded depth field map. Therefore, the size of the target may be determined in a first step at a first distance, particularly a first distance at which the distance measurement device is functional, such as at a distance of 16 cm between the at least one target and the distance measurement device. The image analysis procedure of the at least one recorded image showing the at least one eye of the person may be analyzed for determining the distance of the at least one eye of the person to the measurement device, wherein the size of the target is considered that is determined by evaluating the at least one recorded depth field map at the predetermined distance, particularly the first distance.

The distance measurement device may be using a first coordinate system and a relation of the first coordinate system to a world coordinate system may be known, particularly due to a calibration. The image capturing unit may be using a second coordinate system and wherein a relation of the second coordinate system to a world coordinate system may be known, particularly due to a calibration. As generally used, the term "world coordinate system" refers to a coordinate system with which at least one further coordinate systems is linked, particularly the first coordinate system and/or the second coordinate system. The world coordinate system may be described by orthogonal axes and may be a Cartesian coordinate system.

The person is guided, in a step v), to take a predetermined position of the at least one eye of the person in relation to and/or relative to the measurement device. As used herein, the term "predetermined position" refers to a predefined position, particularly a given location of the at least one eye of the person with respect to the measurement device, specifically the image capturing unit and/or the displayed at least one feature, more specifically the center of the at least one feature and/or the center of the displayed at least one feature. The predetermined position may be taken by moving at least one of: the measurement device; of the eye of the person. The predetermined position may be optimized for recording the at least one image in a manner that the determination of the astigmatism parameter may be optimized. Step v) may be performed before at least one of the steps i), ii), iii) or iv). The at least one position of the at least one eye of the person may be continuously determined, particularly by performing step iii), when the person may be guided to take the predetermined position. As generally used, the term "continuously determined" refers to a measurement and/or determination of the at least one position of the at least one eye that is constantly, permanently, and/or repeatedly performed. A continuous determination may be performed all the time and/or after a predetermined time interval, such as after every 16ms, 33ms, 50ms, 100ms, 200ms.

The person may be guided to take a predetermined position of the at least one eye of the person in relation to the measurement device by at least one of:

- an audible signal;
- a visual signal; or
- a tactile signal.

The respective signal may be given by the measurement device. As used herein, the term "visual signal" refers to a stimulus that is displayed to the at least one eye of the person. The term "audio signal" refers to a stimulus that is perceptible by a sense of hearing of the person. The term "tactile signal" refers to a stimulus that is perceived by a haptic sensation, for example haptic sensations such as, but not limited to, a vibration of the measurement device.

The visual signal may be displayed on the measurement device, particularly the screen comprised by the measurement device. The at least one eye of the person may be further displayed on the screen, wherein the person may be indicated to take the predetermined position in a manner that the displayed at least one eye of the person is to be positioned at a location indicated by the displayed visual signal.

The predetermined position may be defined by a paraxial model, particularly the predetermined position in the plane may be defined by a paraxial model. As used herein, the term "paraxial" refers to an approximation, particularly a small-angle approximation, specifically used in Gaussian optics. In the paraxial model, this approximation is considered.

The paraxial model may be defined by an isosceles triangle having at least two sides of equal length, wherein a first side of equal length may begin at the image capturing unit, particularly a center of the image capturing unit, and may end at the at least one eye of the person, wherein a second side of equal length may begin at the at least one eye of the person and may end at the at least one known feature being displayed on the at least one screen of the at least one screen of the measurement device, particularly a center of the at least one feature. The predetermined position may be defined by a predetermined distance, particularly wherein the predetermined distance is between 10 cm and 25 cm, particularly 13,5 cm.

A determined position of the at least one eye of the person may be compared to the predetermined position by determining a first deviation between the position of the at least one eye of the person and the predetermined position, particularly wherein the image of the at least one reflected feature on the eye of the person, in step b), may be, specifically only, recorded when the first deviation is acceptable in view of a first threshold. The first deviation may be acceptable in view of a first threshold, when the first deviation is below the first threshold. As used herein, the term "first deviation" refers to at least one value that considers a divergence between the position of the at least one eye of the person and the predetermined position, in at least one dimension and/or in at least two dimensions and/or in any dimension.

The orientation of the at least one eye of the person may be aligned, in a step vi), to a predetermined orientation of the at least one eye. As used herein, the term "predetermined orientation" refers to a predefined orientation, particularly a given direction of a line of sight of the at least one eye of the person with respect to the measurement device, specifically the image capturing unit, and/or the displayed at least one feature, particularly the center of the at least one feature, and/or the distance measurement device, wherein the predetermined orientation may be, particularly, optimized for recording the at least one image in a manner that the determination of the astigmatism parameter may be optimized. In the predetermined orientation, the line of sight of the at least one eye of the person may be directed onto a center of an abstract line connecting the image capturing unit, particularly the center of the image capturing unit, and the displayed known feature, particularly a center of the displayed known feature, more particularly on the screen. Step vi) may be performed after step v) but before at least one of the steps i), ii), iii) or iv). In the predetermined orientation the at least one known feature may be reflected in the center of the at least one eye of the person. As used herein, the term "center of the at least one eye of the person" refers to a center of a visual axis on a cornea of the at least one eye of the person.

At least one visual stimulus may be displayed on the screen of the measurement device, wherein the at least one visual stimulus may be configured for aligning the orientation of the at least one eye of the person to the predetermined orientation by causing the person to direct a line of sight of the at least one eye of the person on the at least one visual stimulus. The location at which the at least one visual stimulus displayed on the screen may be determined by considering at least one of:

- a position of the measurement device, particularly a position of at least one of:

  ○ the image capturing unit; or
  ○ the screen, particularly for considering a location of the at least one known feature on the screen; or

- the position of the at least one eye of the person.

The orientation of the at least one eye of the person may be determined, in step vi), wherein the determined orientation of the at least one eye may be compared to the predetermined orientation by determining a second deviation between the orientation of the at least one eye of the person and the predetermined orientation, particularly wherein the image of the at least one reflected feature on the eye of the person may be recorded, specifically only, when the second deviation may be acceptable in view of a second threshold. The second deviation may be acceptable in view of a second threshold, when the second deviation is below the second threshold. As used herein, the term "second deviation" refers to at least one value that considers a divergence between the orientation of the at least one eye of the person and the predetermined orientation.

The location at which the at least one visual stimulus may be displayed, particularly in step e), may be adjusted by considering the at least one image of the at least one reflected feature on the eye of the person, specifically recorded after or when a preceding at least one visual stimulus was displayed, particularly wherein subsequently a further at least one image of the at least one reflected feature on the eye of the person is recorded. As used herein, the term "adjusting", or any grammatical variation thereof, specifically "adjusted", refers to an amendment and/or change of the location at which the at least one visual stimulus is displayed, particularly intended for further optimizing the orientation of the eye of the person.

According to the invention, the at least one known feature is configured for determining at least three different radii of the at least one eye of the person. As used herein, the term "different radius", or any grammatical variation thereof, specifically "different radii", refers to at least one radius related to the surface of the eye of the person. The three different radii may be laying in three different directions, particularly defined by three different angle $\alpha$.

According to the invention, the at least one known feature comprises a plurality of identifiable markings, particularly wherein the at least three different radii are determined by considering the plurality of identifiable markings, particularly in the reflected feature. The identifiable markings may be identifiable points. As used herein, the term "identifiable markings" refers to characteristics of the feature that are recognizable, particularly by using an image analysis procedure. The identifiable markings may be clearly distinguishable from further portion of the feature and/or from a background, specifically comprised by the at least one image of the at least one eye of the person. The at least one known feature comprising the plurality of identifiable markings may be selected from at least one of: a pattern of points; a ring; a donut; a star; or a stochastic feature.

Determining the at least one astigmatism parameter, particularly in step iii), may comprise determining at least three different distances from the at least one image of the at least one reflected feature on the eye of the person, particularly by at least one of:

- identifying the plurality of identifiable markings in the image of the at least one reflected feature of the eye of the person

- determining the at least three different distances between the plurality of identifiable markings.

As used herein, the term "different distances" refers to a plurality of distances that differ particularly in their direction, particularly defined by three different angle $\alpha$. Determining the at least three different distances may comprise considering a plurality of at least one distance of the at least three distances determined from a plurality of the at least one image of the at least one reflected feature, particularly by determining a mean for the at least one distance by considering the plurality of the at least one distance. Alternatively or in addition, determining the at least three different distances may comprise considering a plurality of at least one distance of the at least three distances determined from the same image of the at least one reflected feature, according to the invention by considering different identifiable markings of the at least one reflected feature, particularly by determining a mean for the at least one distance by considering the plurality of the at least one distance.

Determining the at least three different distances from the at least one image may comprise considering the position of the at least one eye of the person, particularly the distance. The at least three different radii of the eye of the person may be determined by considering the at least three different distances between the plurality of identifiable markings in the reflected feature, particularly by further considering the paraxial model.

According to the invention, determining the at least one astigmatism parameter comprises correcting the first deviation between the position of the at least one eye of the person and the predetermined position, particularly by considering correction data comprising correction information. The correction data may comprise information about at least one parameter configured for compensating the first deviation. The correction may be performed by an analytical method, particularly in step iv). The correction data may comprise correction information for a plurality of predetermined first deviations. The correction data may be interpolated in case no predetermined first deviation equals the to be corrected first deviation. Thereby, an interpolated predetermined first deviation may be generated that equals the to be corrected first deviation. The correction data may be determined in a calibration procedure. The correction data may be specific for the type and/or model of the measurement device.

The correction data may comprise information about a function $F$, wherein each one of the at least three different radii $r_{par}^{\propto}$ may be corrected, by considering equation (1)

$$r_{corr}^{\propto} = F^{\propto}\big(x, y, z, r_{par}^{\propto}\big), \qquad\qquad (1)$$

wherein x, y and z may be the position of the at least one eye of the person, wherein $r_{corr}^{\propto}$ may be the corrected radii, wherein $\alpha$ may be an angle describing a direction of the radii.

At least one meridian of the at least one eye of the person may be determined by at least one of the following steps:

- determining at least one normal to the surface of the at least one eye at at least one of the plurality of identifiable markings, particularly by considering at least one of:

   ◦ the location of the at least one feature on the measurement device, particularly the screen of the measurement device;
   ◦ the position of the at least one eye of the person relative to the measurement device;
   ◦ the position of the image capturing unit, particularly the position of the detected at least one feature on the image capturing unit; or
   ◦ a direction of the detected light reflected on the eye of the person;

- using a regression algorithm for optimizing an ellipsoid to at least three different normal to the surface, particularly wherein the regression algorithm optimizes at least one of:

   ◦ an angle of an axis of the ellipsoid;
   ◦ a first meridian of the ellipsoid, particularly wherein the first meridian is a small meridian; or
   ◦ a second meridian of the ellipsoid, particularly wherein the second meridian is a large meridian.

As generally used, the term "ellipsoid" refers to a three dimensional equivalent of an ellipse. An ellipsoid may be obtained from a sphere by performing at least one directional scaling and/or by at least one affine transformation. The ellipsoid may be described by at least one of: an angle of an axis of the ellipsoid; a first meridian of the ellipsoid, particularly wherein the first meridian is a small meridian; or a second meridian of the ellipsoid, particularly wherein the second meridian is a large meridian. As generally used, the term "degree of freedom" refers to a number of independent parameter, such as at least one of: an angle of an axis of the ellipsoid; a first meridian of the ellipsoid, particularly wherein the first meridian is a small meridian; or a second meridian of the ellipsoid, particularly wherein the second meridian is a large meridian.

At least one meridian of the at least one eye of the person may be determined by considering the at least three different radii, particularly by at least one of:

- determining an ellipsoid by using at least one equation, particularly determining at least one of:

  ○ an angle of an axis of the ellipsoid;
  ○ a first meridian of the ellipsoid, particularly wherein the first meridian is a small meridian; or
  o a second meridian of the ellipsoid, particularly wherein the second meridian is a large meridian;

- using a regression considering a second degree parabola to the smallest radius of the at least three radii and a second degree parabola to the largest radius of the at least three radii; or
- using a regression for optimizing an ellipsoid to the at least three different radii, particularly wherein the regression algorithm optimizes at least one of:

  ○ an angle of an axis of the ellipsoid;
  ○ a first meridian of the ellipsoid, particularly wherein the first meridian is a small meridian; or
  ○ a second meridian of the ellipsoid, particularly wherein the second meridian is a large meridian.

As generally used, the term "regression" refers to a statistical analysis tool that aims to determine a relationship between an input and a statistical model to determine optimized output parameters. A regression may be a fit, wherein the term "fit" refers to a process of constructing a mathematical function that has the best fit to a series of data points.

For determining the ellipsoid by using at least one equation, a rotational ellipsoid may be assumed. A rotational ellipsoid may be an ellipsoid that results when an ellipse is rotated. The at least one equation (2) used for determining the ellipsoid may be

$$r_\alpha = \sqrt{\frac{a^2 b^2}{(b^2(\cos\alpha + \gamma)^2 + a^2(\sin\alpha + \gamma)^2)}} \qquad (2)$$

wherein $r_\alpha$ is one of the least three different radii. The rotational ellipsoid may be determined by considering three different equations (2) for three different radii $r_\alpha$, wherein the parameter $a$ is the small meridian, $b$ is the large meridian and $\gamma$ is the angle of the axis of the ellipsoid. The parameters may be determined numerically.

For fitting the second degree parabola, the measured or determined radii may be sorted according to their value. Since the radii are on an ellipse, the small meridian may lie close to the smallest radii. A second degree parabola may be fitted through the smallest radius and the radii laying directly left and directly right of the smallest radii. The vertex of the second degree parabola may then be considered as the small meridian and the location of the vertex may be considered an inclination axis of the ellipsoid. The same may be done with the large meridian, except that it may be fitted around the largest measured radius.

The at least one astigmatism parameter may be determined by considering the at least one meridian. The at least one astigmatism parameter, particularly the cylindrical power CYL, may be determined by considering equation (3)

$$CYL = 0.376 * \frac{b - a}{a * b}, \qquad (3)$$

wherein a is the large meridian and b is the small meridian.

A roll of a head of the person comprising the at least one eye may be considered for determining the at least one astigmatism parameter, particularly for correcting a cylinder axis. As generally used, the term "roll" refers to a rotation around a longitudinal axis, or roll axis, of the head of the person, particularly wherein the longitudinal axis is an axis pointing from the back of the head to the nose of the head. The roll may be different from a yaw axis, or a pitch axis. The roll of the head of the person may be determined by considering a pose of the head of the at least one person, particularly by analyzing a vertical offset between both eyes of the person. Alternatively or in addition, the roll of the head of the person may be determined by evaluating at least one of:

- at least one image of the head of the person, particularly wherein the at least one image of the head of the person is the image of the at least one reflected feature on the eye of the person that is further showing the head of the person; or
- at least one depth field map of the head of the person.

The at least one image of the at least one reflected feature on the eye of the person may be recorded by using an external lens for the at least one measurement device, particularly for the at least one image capturing unit. As used herein, the term "external lens" refers to a lens that is separate from the measurement device. The external lens may be used for changing the focus point of the measurement device and/or the image capturing unit, particularly towards the measurement device and/or the image capturing unit. Thereby, an object, such as the at least one eye of the user may be recorded that is closer to the measurement device. The external lens may be arranged on the measurement device by using a holder comprising the external lens, that is, particularly in a removable manner, attachable to the measurement device.

The input data may comprise measured data, specifically at least one of:

- the at least one image of the at least one reflected feature on the eye of the person; or
- the at least one distance of the at least one eye of the person, and

The person may be a real person. As used herein the term "measured data" refers to data that is determined from at least one measurement in a physical reality that is particularly extern of a simulation. The physical reality may be different from a simulated reality. As used herein the term "real person" refers to a person that is present in the physical reality that is particularly extern of a simulation. The real person is different from a simulated person. The method may comprise actively recording the measured data. As used herein the term "actively determining" or any grammatical variation thereof refers to the method comprising at least one step in which the measured data is determined. Thereby, the method may operate at least one measurement device, particularly the distance measurement device.

The astigmatism parameter of the eye of the person may be at least one of a value related to:

- a cylindrical power; or
- a cylinder axis.

Based on standard ISO 13666:2019 (referred to "Standard" in the following), Section 3.13.7, the term "cylindrical power", usually abbreviated to "cylinder" or "cyl", refers to an algebraic difference between principal powers with power of the principal meridian chosen for reference being subtracted from the other principal power. As based on in the Standard, Section 3.13.8, the term "cylinder axis", usually abbreviated to "cyl axis" or "axis", refers to a direction of the principal meridian of a lens whose vertex power is chosen for reference.

The measurement device may be selected from at least one of:

- a mobile device, specifically at least one of:

    - a smartphone;
    - a wearable, such as a smart watch; or
    - a tablet;

- a personal computer, specifically at least one of

    - a desktop computer; or
    - a laptop; or

- a smart television.

As generally used, the term "mobile device" refers to a mobile electronics device, more specifically to a mobile communication device such as a cell phone or smart phone. Additionally or alternatively, as will be outlined in further detail below, the mobile device may also refer to a tablet computer or another type of portable computer having at least one image capturing unit. As generally used, the term "smartphone" refers to a mobile phone having computer functionalities and connectivity and may, additionally, comprise at least one sensor and/or at least one actuator, for example at least one vibration motor. As generally used, the term "wearable" refers to an electronic device, such as a computer, that is worn on the body of the person, particularly during an intended use of the wearable. As generally used, the term "smart watch" refers to an electronic wristwatch that has computer functionalities and connectivity and may, additionally, comprise at least one sensor and/or at least one actuator, for example at least one vibration motor. As generally used, the term "tablet" refers to a portable, flat touch-screen computer. As generally used, the term "personal computer" refers to a multi-purpose computer whose size, capabilities, and price make it feasible for an individual use. Personal computers are configured for being operated directly by an end user, rather than by a computer expert or a technician. As generally used, the term "desktop computer" refers to a computer in a housing shape suitable for use as a workstation computer on desks. As generally used, the term "laptop" refers to a special type of computer having a screen being movably attached to a housing, wherein the screen can be folded onto the housing. As generally used, the term "smart television" refers to a television having computer functionalities and connectivity.

The distance measurement device may be selected from at least one of:

- a device configured for recording a structured light;
- a device configured for recording a stereo vision;
- a time of flight measurement device; or
- a LiDAR.

As generally used, the term "device configured for recording a structured light" refers to an apparatus that may be an image capturing unit. As generally used, the term "a device configured for recording a stereo vision" refers to an apparatus that may comprise at least two image capturing units, specifically cameras. As generally used, the term "time of flight measurement device" is an apparatus configured for performing a method for measuring at least one distance between the time of flight measurement device and an object, wherein the evaluation of the distance is based on the time difference between the emission of a signal and its return to the sensor, after being reflected by an object. The time of flight measurement device may be configured to perform a direct and/or an indirect measurement. Alternatively or in addition, the time of flight measurement device may be configured to perform a solid state and/or MEMs based indirect time of flight measurement or a flash based camera measurement. As generally used, the term "LiDAR" refers a device configured for determining the at least one distance by targeting an object or a surface with a laser and measuring the time for the reflected light returning to the receiver.

The image capturing unit may be selected from at least one of:

- a camera, particularly a front camera;
- a webcam.

As generally used, the term "camera" refers to an optical device that captures visual images. As generally used, the term "webcam" refers to a small camera that may be placed on or in a monitor, or may be introduced into a computer.

In a further aspect, the present invention relates to a computer program according to claim 9 comprising instructions which, when the program is executed by a computer, cause the computer to carry out the method for determining the at least one astigmatism parameter according to claim 1.

As generally used, the term "computer program" refers to at least one executable instruction for at least one programmable apparatus, specifically a computer, preferably a sequence of executable instructions, for processing and/or solving of at least one function and/or at least one task and/or at least one problem by using at least one programmable apparatus or device, specifically a computer, preferably for performing some or all steps of any one of the methods as described within the present invention. Typically, instructions are combined to a computer program code and/or provided in a programming language. A computer program is typically processed by using a processing device comprised by the at least one computer. For this purpose, the computer program may be running on the computer. The computer program code may be provided on a data storage medium or a separate device such as an optical storage medium, e.g., on a compact disc, directly on a computer or data processing device, or via a network, such as via an in-house network or via internet.

For further details concerning the computer program, a reference may be made to any aspect and/or any definition according to the present invention as disclosed elsewhere herein.

In a further aspect, the present disclosure relates to a field device not forming part of the claimed invention for generating input data for determining at least one astigmatism parameter of at least one eye of a person, wherein the field device is configured to carry out a computer-implemented method for determining at least one astigmatism parameter of at least one eye of a person, wherein the field device is used as a measurement device, comprising the following steps:

a) generating input data configured to comprise

- at least one known feature configured for determining the at least one astigmatism parameter of the at least one eye of the person;
- at least one image of at least one reflected feature in the eye of the person, wherein the at least one image of the at least one reflected feature is generated, in a step i), by displaying the at least one known feature on a measurement device in a manner that the at least one known feature is reflected by the eye of the person; wherein the at least one image of the at least one reflected feature in the eye of the person is recorded, in a step ii), by using the measurement device;
- at least one position of the at least one eye of the person relative to the measurement device; wherein the at least one position of the at least one eye of the person is determined, in a step iii), by using the measurement device;

wherein the input data set is forwarded to a remote device configured for generating outcome data by considering the position of the at least one eye of the person by using a connection interface.

For further details concerning the field device, a reference may be made to any aspect and/or any definition according to the present invention as disclosed elsewhere herein. The field device may be or may be used as the measurement device.

As used herein, the term "field device" refers to a device that is used for generating the input data. The person may have access to the field device and/or the person may use the field device, particularly for generating the input data. Alternatively, a further person may use the field device, particularly for generating the input data. The field device may be operable without a remote device. As used herein, the term "remote device" refers to a device that is used for generating the outcome data. The person may not have access to the field device and/or the person may not or may only indirectly use the field device, particularly for generating the outcome data. The remote device may be owned, used and/or controlled by a third party, for example a company or a further person. The remote device may be operable without the field device. The field device may transmit the input data to a specific remote device depending on at least one circumstance, such as a date, a day, a low load of the specific remote device, particularly in comparison to at least one alternative remote device, low costs for running the specific remote device, particularly in comparison to at least one alternative remote device, and so on. The specific remote device may not be directly selected by the field device but rather a further device may specify to which specific remote device the input data may be transmitted from the field device. The generation of the outcome data may involve a use of several different entities of the remote device. At least one entity may generate intermediate data and transmit the intermediate data by using a connection interface to at least one further entity. The outcome data may be transmitted from the remote device to the field device, particularly by using a data carrier signal carrying the outcome data. Thereby, the person may be able to analyze the outcome data, specifically the at least one astigmatism parameter of the eye of the person.

As generally used, the term "connection interface" refers an item or an element forming a boundary configured for transmitting information or data, particularly input data, or outcome data. **In** particular, the connection interface may be configured for transmitting information from a computational device, e.g. a computer, such as to forward input data or outcome data, e.g. onto another device. Additionally, or alternatively, the connection interface may be configured for transmitting information onto a computational device, e.g. onto a computer, such as to receive information. The connection interface may specifically be configured for transmitting or exchanging information. **In** particular, the connection interface may provide a data transfer connection, e.g. Bluetooth, NFC, or inductive coupling. As an example, the connection interface may be or may comprise at least one port comprising one or more of a network or internet port, a USB-port, and a disk drive.

The field device may be at least one of:

- a mobile device, specifically at least one of:

  ∘ a smartphone;
  ∘ a wearable, such as a smart watch; or
  ∘ a tablet;

- a personal computer, specifically at least one of

  ∘ a desktop computer; or
  ∘ a laptop; or

- a smart television.

In a further aspect, the present invention relates to a remote device for generating outcome data for determining at least one astigmatism parameter of at least one eye of a person according to claim 10, wherein the remote device is configured to carry out a computer-implemented method for determining at least one astigmatism parameter of at least one eye of a person, wherein the remote device is receiving input data provided by a field device by using a connection interface, wherein the method is comprising, inter alia, the following step:

b) generating outcome data, particularly by using a processing device, configured to comprise

- at least one astigmatism parameter of the eye of the person; wherein the at least one astigmatism parameter of the eye of the person is determined, in a step iv), by comparing the at least one known feature to the at least one reflected feature comprised in the at least one recorded image of the at least one reflected feature in the eye of the person, wherein the position of the at least one eye of the person is considered when the at least one astigmatism parameter of the eye of the person is determined.

For further details concerning the remote device, a reference may be made to any aspect and/or any definition according to the present invention as disclosed elsewhere herein.

As generally used, the term "processing device" refers to an arbitrary logic circuitry configured for performing basic operations of a computer or system, and/or, generally, to a device which is configured for performing calculations or logic operations. Herein, the processing device may be a single device. As an alternative, the processing device may comprise a plurality of elements which are located on more than a single location, wherein at least two elements located on at least two different locations are configured to communicate with each other by using at least one connection interface, especially at least one connection interface as described elsewhere herein in more detail. In particular, the processing unit may be configured for processing basic instructions that drive the computer or system. As an example, the processing unit may comprise at least one arithmetic logic unit (ALU), at least one floating-point unit (FPU), such as a math co-processor or a numeric coprocessor, a plurality of registers, specifically registers configured for supplying operands to the ALU and storing results of operations, and a memory, such as an L1 and L2 cache memory. In particular, the processing unit may be a multi-core processor. Specifically, the processing unit may be or may comprise a central processing unit (CPU). Additionally, or alternatively, the processing unit may be or may comprise a microprocessor, thus specifically the processing unit's elements may be contained in one single integrated circuitry (IC) chip. Additionally, or alternatively, the processing unit may be or may comprise one or more application-specific integrated circuits (ASICs) and/or one or more field-programmable gate arrays (FPGAs). The processing unit specifically may be configured, such as by software programming, for performing one or more evaluation operations.

The remote device may be at least one of:

- a server; or
- a cloud server.

As generally used, the term "server" refers to device for performing at least one task for a further device connected to the server for transmitting data, particularly connected by a network such as the internet or a private network. As generally used, the term "cloud server" refers to a server that is accessible via a public network such as the internet. A cloud server may particularly be owned by a third party and offer third party service such as evaluating the input data for generating the outcome data and thereby determining the at least one astigmatism parameter of the eye of the person.

The connection interface may be selected from at least one of:

- a network interface controller; or
- a transmitter.

As generally used, the term "network interface controller" refers to a computer hardware component that connects a computer to a computer network. As generally used, the term "transmitter" refers to an electronic device, which produces electromagnetic waves with an antenna.

In a further aspect, the present disclosure relates to a data carrier signal not forming part of the claimed invention carrying the outcome data generated by a method for determining the at least one astigmatism parameter as described elsewhere herein. For further details concerning the data carrier signal, a reference may be made to any aspect and/or any definition according to the present invention as disclosed elsewhere herein. As generally used, the term "data carrier signal" refers to an electronic signal comprising information. The data carrier signal may be provided on a computer-readable storage medium. As used herein, the term "computer-readable storage medium" specifically may refer to a non-transitory data storage means, such as a hardware storage medium having stored thereon computer-executable instructions.

In a further aspect, the present invention relates to a determining device according to claim 11 for determining at least one astigmatism parameter of an eye of a person, wherein the device is configured to carry out a computer-implemented method for determining at least one astigmatism parameter of an eye of a person, wherein the determining device is used as a measurement device, wherein the method is comprising, inter alia, the following steps:

a) generating input data configured to comprise

- at least one known feature configured for determining the at least one astigmatism parameter of the at least one eye of the person;
- at least one image of at least one reflected feature in the eye of the person, wherein the at least one image of the at least one reflected feature is generated, in a step i), by displaying the at least one known feature on a measurement device in a manner that the at least one known feature is reflected by the eye of the person;

wherein the at least one image of the at least one reflected feature in the eye of the person is recorded, in a step ii), by using the measurement device;

- at least one position of the at least one eye of the person relative to the measurement device; wherein the at least one position of the at least one eye of the person is determined, in a step iii), by using the measurement device;

b) generating outcome data, particularly by using a processing device, configured to comprise

- at least one astigmatism parameter of the eye of the person; wherein the at least one astigmatism parameter of the eye of the person is determined, in a step iv), by comparing the at least one known feature to the at least one reflected feature comprised in the at least one recorded image of the at least one reflected feature in the eye of the person, wherein the position of the at least one eye of the person is considered when the at least one astigmatism parameter of the eye of the person is determined.

For further details concerning the determining device, a reference may be made to any aspect and/or any definition according to the present invention as disclosed elsewhere herein. The determining device may be or may be used as the measurement device.

The determining device may be at least one of:

- a mobile device, specifically at least one of:

    ◦ a smartphone;
    ◦ a wearable, such as a smart watch; or
    ◦ a tablet;

- a personal computer, specifically at least one of

    ◦ a desktop computer; or
    ◦ a laptop; or

- a smart television.

The device may be configured to carry out a computer-implemented method for determining at least one astigmatism parameter of an eye of a person as described elsewhere herein.

In a further aspect, the present invention relates to a method for producing a geometrical model of at least one spectacle lens for manufacturing of the at least one spectacle lens according to claim 12, wherein producing the geometrical model comprises, inter alia,

- producing a geometrical model of at least one spectacle lens for at least one eye of a person by using data related to at least one refractive value; and
- determining the data related to the at least one refractive value by carrying out a computer-implemented method for determining at least one astigmatism parameter of at least one eye of a person in a vision testing procedure, wherein the vision testing procedure comprises at least one test cycle, preferably at least two subsequent test cycles, wherein the test cycle comprises at least the following steps:

    a) generating input data configured to comprise

    - at least one known feature configured for determining the at least one astigmatism parameter of the at least one eye of the person;
    - at least one image of at least one reflected feature in the eye of the person, wherein the at least one image of the at least one reflected feature is generated, in a step i), by displaying the at least one known feature on a measurement device in a manner that the at least one known feature is reflected by the eye of the person; wherein the at least one image of the at least one reflected feature in the eye of the person is recorded, in a step ii), by using the measurement device;
    - at least one position of the at least one eye of the person relative to the measurement device; wherein the at least one position of the at least one eye of the person is determined, in a step iii), by using the measurement device;

    b) generating outcome data, particularly by using a processing device, configured to comprise

- at least one astigmatism parameter of the eye of the person; wherein the at least one astigmatism parameter of the eye of the person is determined, in a step iv), by comparing the at least one known feature to the at least one reflected feature comprised in the at least one recorded image of the at least one reflected feature in the eye of the person, wherein the position of the at least one eye of the person is considered when the at least one astigmatism parameter of the eye of the person is determined.

As used herein, the term "refractive value" refers to a characteristic chosen to counteract an astigmatism parameter as defined above in more detail. The characteristic may be a characteristic of the geometrical model of at least one spectacle lens or of the at least one spectacle lens. As used herein, the term "vision testing procedure" refers to a procedure in which the at least one refractive error, such as a astigmatism parameter, of an eye of a person is determined. The at least one vision testing procedure may comprise the steps of the computer-implemented method for determining at least one astigmatism parameter of the eye of the person as described elsewhere herein.

For further details concerning the method for producing a geometrical model, a reference may be made to any aspect and/or any definition according to the present invention as disclosed elsewhere herein.

In a further aspect, the present invention relates to a method for producing at least one spectacle lens according to claim 13, wherein the at least one spectacle lens is manufactured by processing at least one lens blank by using the produced geometrical model of the at least one spectacle lens according to claim 12.

For further details concerning the method for producing at least one spectacle lens, a reference may be made to any aspect and/or any definition according to the present invention as disclosed elsewhere herein.

In a further aspect, the present invention relates to a use of an external lens for performing the method for determining at least one astigmatism parameter according to claim 14, wherein the external lens is arranged on the at least one image capturing unit. For further details concerning the use of an external lens, a reference may be made to any aspect and/or any definition according to the present invention as disclosed elsewhere herein.

Referring to the computer-implemented aspects of the invention, one or more of the method steps or even all of the method steps of the method as disclosed herein may be performed by using a computer or a computer network. Thus, generally, any of the method steps including provision and/or manipulation of data may be performed by using the computer or the computer network. Generally, these method steps may include any of the method steps, typically except for method steps requiring manual work, such as certain aspects of performing the actual measurements.

Various embodiments may be conceived for implementing the methods according to the present invention. According to a first embodiment, all method steps may be performed by using a single processing device, such as a computer, especially a virtual reality headset, an augmented reality system, a desktop computer, a television set, smart glasses, or a mobile communication device, specifically a smartphone. In this embodiment, the single processing device may be configured to exclusively perform at least one computer program, in particular at least one line of computer program code configured to execute at least one algorithm, as used in at least one of the methods according to the present invention. Herein, the computer program as executed on the single processing device may comprise all instructions causing the computer to carry out at least one of the methods according to the present invention. Alternatively, or in addition, at least one method step may be performed by using at least one remote processing device, especially selected from at least one of a server or a cloud server, which is not located at the site of the person when executing the at least one method step. In this further embodiment, the computer program may comprise at least one remote portion to be executed by the at least one remote processing device to carry out the at least one method step. Further, the computer program may comprise at least one interface configured to forward to and/or receive data from the at least one remote portion of the computer program.

With respect to the prior art, the device exhibits the following advantages.

The invention provides fast, easy, versatile, reliable, and accurate approach for determining at least one astigmatism parameter of at least one eye of a person.

By considering the position of the eye of the person, the measurement of the astigmatism parameter may improve in repeatability and/or accuracy compared to methods in which the astigmatism parameter is determined, but the position of the eye of the person is not considered. Further, self-testing by the user may be possible by using typical consumer devices, such as smartphones. As a result, the astigmatism measurements are easy accessible due to the large availability of the typical consumer devices and the known usability of these typical consumer devices for the end user.

As used herein, the terms "have", "comprise" or "include" or any arbitrary grammatical variation thereof are used in a non-exclusive way. Thus, these terms may refer to both a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In most cases, when referring to the respective feature or element, the expressions "at least one" or "one or more" will not be repeated, notwithstanding the fact that the respective feature or element may be present once or more than once.

As further used herein, the terms "preferably", "more preferably", "particularly", "more particularly", or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in this way with other features of the invention.

Short description of the Figures

Further optional features and embodiments of the present invention are disclosed in more detail in the subsequent description. Therein, the respective optional features may be implemented in an isolated fashion as well as in any arbitrary feasible combination, as the skilled person will realize. It is emphasized here that the scope of the invention is not restricted by the preferred embodiments.
In the Figures:

Figure 1   illustrates an exemplarily determining device for determining at least one astigmatism parameter of an eye of a person used as a measurement device;

Figure 2   illustrates an exemplarily computer-implemented method for determining the at least one astigmatism parameter of an eye of a person;

Figure 3   illustrates an exemplary known feature and an image comprising an exemplary reflected feature recorded by the determining device;

Figure 4   illustrates an exemplary predetermined position of the at least one eye of the person relative to the determining device;

Figure 5   illustrates a further exemplary reflected feature showing at least three distances considered for determining the at least one astigmatism parameter;

Figure 6   illustrates an exemplary field device and an exemplary remote device; and

Figure 7   illustrates a method for producing a geometrical model and method for producing at least one spectacle lens.

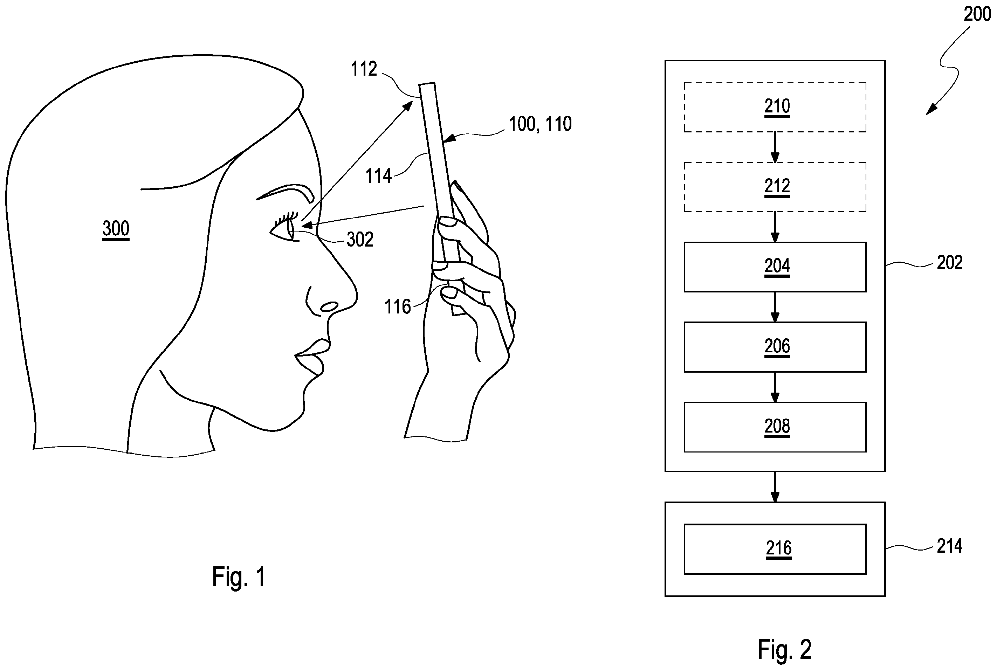

Fig. 1 shows a determining device 100 for determining at least one astigmatism parameter of an eye 302 of a person 300. The determining device 100 is used as a measurement device 110 in a computer-implemented method for determining the at least one astigmatism parameter of an eye of a person. The astigmatism parameter of the eye 302 of the person 300 may be at least one of a value related to: a cylindrical power; or a cylinder axis.
The exemplary determining device 100 is a mobile device, specifically a smartphone. Alternatively or in addition, the mobile device may be a wearable, such as a smart watch; and/or a tablet. Alternatively or in addition, the determining device 100 may be a personal computer, specifically a desktop computer; and/or a laptop. Alternatively or in addition, the determining device 100 may be a smart television.
The determining device 100 may be configured to carry out a computer-implemented method 200 for determining at least one astigmatism parameter of an eye 302 of a person 300. An exemplary computer-implemented method 200 for determining the at least one astigmatism parameter of an eye 302 of a person 300 is depicted in Fig. 2. The exemplary computer-implemented method 200 for determining the at least one astigmatism parameter of at least one eye 302 of a person 300 is comprising the following steps:

a) generating input data 202 configured to comprise

- at least one known feature 118 configured for determining the at least one astigmatism parameter of the at least one eye 302 of the person 300;
- at least one image 120 of at least one reflected feature 122 in the eye 302 of the person 300, wherein the at least one image 120 of the at least one reflected feature 122 is generated, in a step i) 204, by displaying the at least one known feature 118 on a measurement device 110 in a manner that the at least one known feature 118 is reflected by the eye 302 of the person 300; wherein the at least one image 120 of the at least one reflected feature 122 in the eye 302 of the person 300 is recorded, in a step ii) 206, by using the measurement device 110;
- at least one position of the at least one eye 302 of the person 300 relative to the measurement device 110; wherein the at least one position of the at least one eye 302 of the person 300 is determined, in a step iii) 208, by using the measurement device 110;

b) generating outcome data 214, particularly by using a processing device, configured to comprise

- at least one astigmatism parameter of the eye 302 of the person 300; wherein the at least one astigmatism parameter of the eye 302 of the person 300 is determined, in a step iv) 216, by comparing the at least one known feature 118 to the at least one reflected feature 122 comprised in the at least one recorded image 120 of the at least one reflected feature 122 in the eye 302 of the person 300, wherein the position of the at least one eye 302 of the person 300 is considered when the at least one astigmatism parameter of the eye 302 of the person 300 is determined.

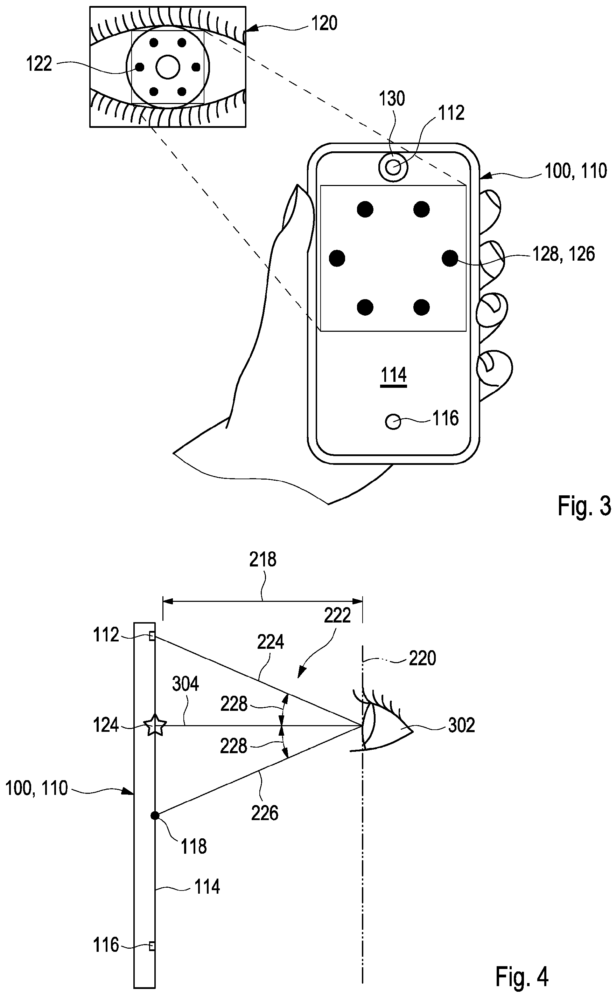

The exemplary determining device 100 illustrated in Fig. 1 is used as the measurement device 110. An exemplary at least one known feature 118 being displayed on the screen 114 and an exemplary image 120 of the at least one reflected feature 122 on the eye 302 of the person 300 are illustrated in Fig.3.

The at least one known feature 118 may be displayed, in step i), on a screen 114 comprised by the measurement device 110 for projecting the at least one feature on the at least one eye 302 of the person 300, particularly onto the eye 302 of the person 300. The image 120 of the at least one reflected feature 122 on the eye 302 of the person 300 may be recorded, in step ii), by using an image capturing unit 112 comprised by the measurement device 110. Again, Fig. 3 illustrates an exemplary least one known feature 118 being displayed on a screen 114 and an image 120 comprising an exemplary reflected feature 122 recorded by using the image capturing unit 112 of the measurement device 110 and/or the determining device 100, respectively. The dotted lines illustrate that the at least one reflected feature 122 is generated by the at least one known feature 118.

As exemplarily illustrated in Fig. 4, determining the at least one position of the eye 302 of the person 300, in step iii), may comprise determining a distance 218 of the at least one eye 302 of the person 300 to the measurement device 110, particularly by using the image capturing unit 112 and/or a distance measurement device 116 (both depicted in Fig. 1 and 3). Determining the at least one position of the eye 302 of the person 300, in step iii), may comprise determining a position of the eye 302 of the person 300 in a plane 220, particularly wherein the plane 220 is perpendicular to the distance 218, particularly by using the image capturing unit 112 and/or the distance measurement device 116. Fig. 4 illustrates a side view of the plane 220.

The distance measurement device 116 may be selected from at least one of: a device configured for recording a structured light; a device configured for recording a stereo vision; a direct and/or indirect Time of Flight measurement device; a LiDAR; or a solid state and/or MEMs based indirect time of flight measurement device. The image capturing unit 112 may be selected from at least one of: a camera, particularly a front camera, specifically of a smartphone; or a webcam.

The at least one position of the at least one eye 302 of the person 300 in relation to the measurement device 110 may be determined directly before, or when the image 120 of the at least one reflected feature on the eye 302 of the person 300 is recorded.

Determining the distance 218 of the at least one eye 302 of the person 300 to the measurement device 110 may comprise at least one of the following steps:

- recording at least one depth field map by using the measurement device 110 , particularly a distance measurement device 116 comprised by the measurement device 110;
- determining the location of the at least one eye 302 of the person 300 in the at least one depth field map, particularly by at least one of:

  ∘ using at least one image 120 analysis procedure on the recorded at least one depth field map; or
  ∘ comparing the recorded at least one depth field map to at least one recorded image 120 showing the at least one eye 302 of the person 300 at the time the at least one depth field map is recorded, wherein the position of the at least one eye 302 of the person 300 in the at least one recorded image 120 showing the at least one eye 302 of the person 300 is determined by using at least on image 120 analysis procedure;

- determining the distance 218 of the at least one eye 302 of the person 300 from the recorded at least one depth field map by considering the determined location of the at least one eye 302 of the person 300 in the at least one depth field map.

Alternatively or in addition, determining the distance 218 of the at least one eye 302 of the person 300 to the measurement device 110 may comprise performing image 120 processing of the at least one recorded image 120 showing the at least one eye 302 of the person 300, particularly by at least one of the following steps:

- considering at least one target having a known target size in at least one recorded image 120 showing the at least one eye 302 of the person 300; particularly wherein the at least one target is selected from at least one of:

  o a credit card; or
  ○ a property of the at least one eye 302 of the person 300, specifically an iris of the at least one eye 302 of the person 300;

- determining the size of the at least one target in the at least one recorded image 120 showing the at least one eye 302 of the person 300, particularly by using at least one image 120 analysis procedure;
- determining the distance 218 by considering the size of the at least one target, particularly by performing a triangulation procedure.

The size of the at least one target at a predetermined distance 218 may be determined by considering at least one recorded depth field map.

The distance measurement device 116 may be using a first coordinate system and a relation of the first coordinate system to a world coordinate system may be known, particularly due to a calibration. The image capturing unit 112 may be using a second coordinate system and a relation of the second coordinate system to a world coordinate system may be known, particularly due to a calibration.

The person 300 is guided, in a step v) 210, to take a predetermined position of the at least one eye 302 of the person 300 in relation and/or relative to the measurement device 110. The predetermined position is illustrated in Fig. 4. Step v) may be performed before at least one of the steps i), ii), iii) or iv). The at least one position of the at least one eye 302 of the person 300 may be continuously determined, particularly by performing step iii), when the person 300 is guided to take the predetermined position.

The person 300 may be guided to take a predetermined position of the at least one eye 302 of the person 300 in relation to and/or relative to the measurement device 110 by an audible signal; and/or a visual signal; and/or a tactile signal. The visual signal may be displayed on the measurement device 110, particularly the screen 114 comprised by the measurement device 110. The at least one eye 302 of the person 300 may be further displayed on the screen 114, wherein the person 300 may be indicated to take the predetermined position in a manner that the displayed at least one eye 302 of the person 300 is to be positioned at a location indicated by the displayed visual signal.

The predetermined position may be defined by a paraxial model, particularly the predetermined position in the plane 220 may be defined by the paraxial model. The paraxial model may be defined by an isosceles triangle 222 having at least two sides 224, 226 of equal length, wherein a first side 224 of equal length begins at the image capturing unit 112, particularly a center of the image capturing unit 112, and ends at the at least one eye 302 of the person 300, wherein a second side 226 of equal length begins at the at least one eye 302 of the person 300 and ends at the at least one known feature 118 being displayed on the at least one screen 114 of the at least one screen 114 of the measurement device 110, particularly a center of the at least one feature. The predetermined position may be defined by a predetermined distance 218, particularly wherein the predetermined distance 218 is between 10 cm and 25 cm, more particularly 13,5 cm.

A determined position of the at least one eye 302 of the person 300 may be compared to the predetermined position by determining a first deviation between the position of the at least one eye 302 of the person 300 and the predetermined position, particularly wherein the image 120 of the at least one reflected feature on the eye 302 of the person 300, in step b), may be, specifically only, recorded when the first deviation is acceptable in view of a first threshold.

The orientation of the at least one eye 302 of the person 300 may be aligned, in a step vi) 212, to a predetermined orientation of the at least one eye 302. Step vi) may be performed after step v) but before at least one of the steps i), ii), iii) or iv). In the predetermined orientation the at least one known feature 118 may be reflected on the center of the at least one eye 302 of the person 300. At least one visual stimulus 124 may be displayed on the screen 114 of the measurement device 110, wherein the at least one visual stimulus 124 may be configured for aligning the orientation of the at least one eye 302 of the person 300 to the predetermined orientation by causing the person 300 to direct a line of sight 304 of the at least one eye 302 of the person 300 on the at least one visual stimulus 124, as exemplarily illustrated in Fig. 4. In the predetermined orientation a first angle 228 between the first side 224 and the line of sight 304 may be equal to a second angle 228 between the second side 226 and the line of sight 304.

The location at which the at least one visual stimulus 124 may be displayed on the screen 114 may be determined by considering at least one of:

- a position of the measurement device 110, particularly a position of at least one of:

  ○ the image capturing unit 112; or
  ○ the screen 114, particularly for considering a location of the at least one known feature 118 on the screen 114; or

- the position of the at least one eye 302 of the person 300.

The orientation of the at least one eye 302 of the person 300 may be determined, in step vi), wherein the determined orientation of the at least one eye 302 may be compared to the predetermined orientation by determining a second deviation between the orientation of the at least one eye 302 of the person 300 and the predetermined orientation, particularly wherein the image 120 of the at least one reflected feature on the eye 302 of the person 300 is recorded, specifically only, when the second deviation is acceptable in view of a second threshold.

The location at which the at least one visual stimulus 124 may be displayed, in step v) 210, may be adjusted by considering the at least one image 120 of the at least one reflected feature 122 on the eye 302 of the person 300, specifically recorded after or when a preceding at least one visual stimulus 124 was displayed, particularly wherein subsequently a further at least one image 120 of the at least one reflected feature 122 on the eye 302 of the person 300 is recorded.

The at least one known feature 118 may be configured for determining at least three different radii of the at least one eye 302 of the person 300. The at least one known feature 118 may comprise a plurality of identifiable markings 126 (as illustrated in Fig. 3), particularly wherein the at least three different radii are determined by considering the plurality of identifiable markings 126, particularly in the reflected feature 122. The at least one known feature 118 comprising the plurality of identifiable markings 126 may be selected from at least one of: a pattern of points; a ring; a donut; a star; or a stochastic feature

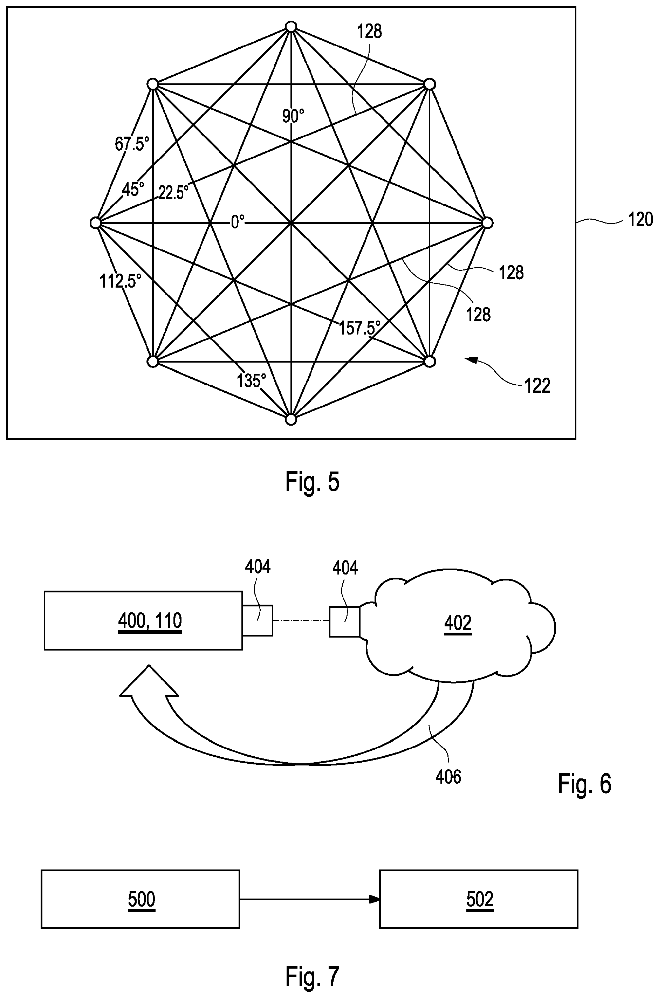

As illustrated in Fig. 5, determining the at least one astigmatism parameter, in step iii), may comprise determining at least three different distances 128 from the at least one image 120 of the at least one reflected feature on the eye 302 of the person 300, particularly by at least one of:

- identifying the plurality of identifiable markings 126 in the image 120 of the at least one reflected feature of the eye 302 of the person 300
- determining the at least three different distances 128 between the plurality of identifiable markings 126.

Determining the at least three different distances 128 may comprise considering a plurality of at least one distance 218 of the at least three distances 218 determined from a plurality of the at least one image 120 of the at least one reflected feature 122, particularly by determining a mean for the at least one distance 218 by considering the plurality of the at least one distance 218. Determining the at least three different distances 128 may comprise considering a plurality of at least one distance 218 of the at least three distances 218 determined from the same image 120 of the at least one reflected feature 122, specifically by considering different identifiable markings 126 of the at least one reflected feature 122, particularly by determining a mean for the at least one distance 218 by considering the plurality of the at least one distance 218. Determining the at least three different distances 128 from the at least one image 120 may comprise considering the position of the at least one eye 302 of the person 300, particularly the distance 218. The at least three different radii of the eye 302 of the person 300 may be determined by considering the at least three different distances 128 between the plurality of identifiable markings 126 in the reflected feature 122, particularly by further considering the paraxial model.

Determining the at least one astigmatism parameter may comprise correcting the first deviation between the position of the at least one eye 302 of the person 300 and the predetermined position, particularly by considering correction data comprising correction information. The correction data may comprise information about at least one parameter configured for compensating the first deviation. The correction data may be determined in a calibration procedure. The correction data may be specific for the type or model of the measurement device 110.

The correction data may comprise information about a function $F$, wherein each one of the at least three different radii $r_{par}^{\propto}$ may be corrected, by considering equation (1)

$$r_{corr}^{\propto} = F^{\propto}\big(x, y, z, r_{par}^{\propto}\big), \qquad (1)$$

wherein x, y and z are the position of the at least one eye 302 of the person 300, wherein $r_{corr}^{\propto}$ is the corrected radii, wherein $\alpha$ is an angle of the radii. In Fig. 5, the depicted angles may be considered as the angle $\alpha$.

At least one meridian of the at least one eye 302 of the person 300 may be determined by at least one of the following steps:

- determining at least one normal to the surface of the at least one eye 302 at at least one of the plurality of identifiable markings 126, particularly by considering at least one of:

  ◦ the location of the at least one feature on the measurement device 110, particularly the screen 114 of the measurement device 110;
  ◦ the position of the at least one eye 302 of the person 300 relative to the measurement device 110;
  ◦ the position of the image capturing unit 112, particularly the position of the detected at least one feature on the image capturing unit 112; or
  ◦ a direction of the detected light reflected on the eye 302 of the person 300;

- using a regression algorithm for optimizing an ellipsoid to at least three different normal to the surface, particularly wherein the regression algorithm optimizes at least one of:

  ◦ an angle of an axis of the ellipsoid;
  ◦ a first meridian of the ellipsoid, particularly wherein the first meridian is a small meridian; or
  ◦ a second meridian of the ellipsoid, particularly wherein the second meridian is a large meridian.

Alternatively or in addition, at least one meridian of the at least one eye 302 of the person 300 may be determined by considering the at least three different radii, particularly by at least one of:

- determining an ellipsoid by using at least one equation, particularly determining at least one of:

  ◦ an angle of an axis of the ellipsoid;
  ◦ a first meridian of the ellipsoid, particularly wherein the first meridian is a small meridian; or
  ◦ a second meridian of the ellipsoid, particularly wherein the second meridian is a large meridian;

- using a regression considering a second degree parabola to the smallest radius of the at least three radii and a second degree parabola to the largest radius of the at least three radii; or
- using a regression for optimizing an ellipsoid to the at least three different radii, particularly wherein the regression algorithm optimizes at least one of:

  ◦ an angle of an axis of the ellipsoid;
  ◦ a first meridian of the ellipsoid, particularly wherein the first meridian is a small meridian; or
  ◦ a second meridian of the ellipsoid, particularly wherein the second meridian is a large meridian.

The at least one astigmatisms parameter may be determined by considering the at least one meridian.
A roll of a head of the person 300 comprising the at least one eye 302 may be considered for determining the at least one astigmatism parameter, particularly for correcting a cylinder axis. The roll of the head of the person 300 may be determined by considering a pose of the head of the at least one person 300, particularly by analyzing a vertical offset between both eye 302s of the person 300. The roll of the head of the person 300 may be determined by evaluating at least one of:

- at least one image 120 of the head of the person 300, particularly wherein the at least one image 120 of the head of the person 300 is the image 120 of the at least one reflected feature 122 on the eye 302 of the person 300 that is further showing the head of the person 300; or
- at least one depth field map of the head of the person 300.

The at least one image 120 of the at least one known feature 118 on the eye 302 of the person 300 may be recorded by using an external lens 130 (depicted in Fig. 3) for the at least one measurement device 110, particularly the at least one image capturing unit 112.
In Fig. 6 an exemplary field device 400 for generating input data 202 for determining at least one astigmatism parameter of at least one eye 302 of a person 300 is illustrated. The field device 400 is configured to carry out a computer-implemented method 200 for determining at least one astigmatism parameter of at least one eye 302 of a person 300, wherein the field device 400 is used as a measurement device 110, comprising the following steps:

  a) generating input data 202 configured to comprise

- at least one known feature 118 configured for determining the at least one astigmatism parameter of the at least one eye 302 of the person 300;
- at least one image 120 of at least one reflected feature 122 in the eye 302 of the person 300, wherein the at least one image 120 of the at least one reflected feature 122 is generated, in a step i) 204, by displaying the at least one reflected feature 122 on a measurement device 110 in a manner that the at least one known feature 118 is reflected by the eye 302 of the person 300; wherein the at least one image 120 of the at least one reflected feature 122 in the eye 302 of the person 300 is recorded, in a step ii) 206, by using the measurement device 110;

- at least one position of the at least one eye 302 of the person 300 relative to the measurement device 110; wherein the at least one position of the at least one eye 302 of the person 300 is determined, in a step iii) 208, by using the measurement device 110;

wherein the input data set is forwarded to a remote device 402 configured for generating outcome data 214 by considering the position of the at least one eye 302 of the person 300 by using a connection interface 404.

The field device 400 is at least one of:

- a mobile device, specifically at least one of:

  o a smartphone;
  ∘ a wearable, such as a smart watch; or
  ∘ a tablet;

- a personal computer, specifically at least one of

  ∘ a desktop computer; or
  ∘ a laptop; or

- a smart television.

A remote device 402 for generating outcome data 214 for determining at least one astigmatism parameter of at least one eye 302 of a person 300 is further illustrated in Fig. 6. The remote device 402 is configured to carry out a computer-implemented method 200 for determining at least one astigmatism parameter of at least one eye 302 of a person 300, wherein the remote device 402 is receiving input data 202 provided by a field device 400 by using a connection interface 404, wherein the method is comprising the following step:

b) generating outcome data 214, particularly by using a processing device, configured to comprise

- at least one astigmatism parameter of the eye 302 of the person 300; wherein the at least one astigmatism parameter of the eye 302 of the person 300 is determined, in a step iv) 216, by comparing at least one known feature 118 comprised by the input data 202 to at least one reflected feature 122 comprised in at least one recorded image 120 of the at least one reflected feature 122 comprised by the input data 202 in the eye 302 of the person 300, wherein a position of the at least one eye 302 of the person 300 comprised by the input data 202 is considered when the at least one astigmatism parameter of the eye 302 of the person 300 is determined.

The remote device 402 may be a server; and/or a cloud server.

The connection interface 404 may be selected from at least one of: a network interface controller; or a transmitter. A data carrier signal 406 may be carrying the outcome data, particularly for sending the outcome data to the field device.

As exemplarily illustrated in Fig. 7, a method for producing a geometrical model 500 of at least one spectacle lens for manufacturing of the at least one spectacle lens, wherein producing the geometrical model comprises

- producing a geometrical model of at least one spectacle lens for at least one eye 302 of a person 300 by using data related to at least one refractive value; and
- determining the data related to the at least one refractive value by carrying out a computer-implemented method 200 for determining at least one astigmatism parameter of at least one eye 302 of a person 300, comprising the following steps:

  a) generating input data 202 configured to comprise

  - at least one known feature 118 configured for determining the at least one astigmatism parameter of the at least one eye 302 of the person 300;
  - at least one image 120 of at least one reflected feature 122 in the eye 302 of the person 300, wherein the at least one image 120 of the at least one reflected feature 122 is generated, in a step i) 204, by displaying the at least one known feature 118 on a measurement device 110 in a manner that the at least one known feature 118 is reflected by the eye 302 of the person 300; wherein the at least one image 120 of the at least one reflected feature 122 in the eye 302 of the person 300 is recorded, in a step ii) 206, by using the measurement device 110;

- at least one position of the at least one eye 302 of the person 300 relative to the measurement device 110; wherein the at least one position of the at least one eye 302 of the person 300 is determined, in a step iii) 208, by using the measurement device 110;

b) generating outcome data 214 by using a processing device configured to comprise

- at least one astigmatism parameter of the eye 302 of the person 300; wherein the at least one astigmatism parameter of the eye 302 of the person 300 is determined, in a step iv) 216, by comparing the at least one known feature 118 to the at least one reflected feature 122 comprised in the at least one recorded image 120 of the at least one reflected feature 122 in the eye 302 of the person 300; wherein the position of the at least one eye 302 of the person 300 is considered when the at least one astigmatism parameter of the eye 302 of the person 300 is determined.

As further exemplarily illustrated in Fig. 7, a method for producing at least one spectacle lens 502, wherein the at least one spectacle lens is manufactured by processing at least one lens blank by using the produced geometrical model of the at least one spectacle lens.

List of Reference Signs

| 100 | determining device |
|---|---|
| 110 | measurement device |
| 112 | image capturing unit |
| 114 | screen |
| 116 | distance measurement device |
| 118 | known feature |
| 120 | image |
| 122 | reflected feature |
| 124 | visual stimulus |
| 126 | identifiable markings |
| 128 | different distances |
| 130 | external lens |
| 200 | computer-implemented method for determining at least one astigmatism parameter |
| 202 | step a): generating input data |
| 204 | step i): displaying the at least one known feature |
| 206 | step ii): recording reflected feature |
| 208 | step iii): determining position of the at least one eye |
| 210 | step v): guiding to predetermined position of the at least one eye |
| 212 | step vi): aligning to predetermined orientation of the at least one eye |
| 214 | step b): generating outcome data |
| 216 | step iv): determining astigmatism parameter |
| 218 | distance |
| 220 | plane |
| 222 | isosceles triangle |
| 224 | first side |
| 226 | second side |
| 228 | angle |
| 300 | person |
| 302 | eye |
| 304 | line of sight |
| 400 | field device |
| 402 | remote device |
| 404 | connection interface |
| 406 | data carrier signal |
| 500 | method for producing a geometrical model |
| 502 | method for producing at least one spectacle lens |

**Claims**

1.  A computer-implemented method (200) for determining at least one astigmatism parameter of at least one eye (302) of a person (300), the method comprising the following steps:

    a) generating input data (202) configured to comprise

    - at least one known feature (118) configured for determining the at least one astigmatism parameter of the at least one eye (302) of the person (300);
    - at least one image (120) of at least one reflected feature (122) in the eye (302) of the person (300), wherein the at least one image (120) of the at least one reflected feature (122) is generated by displaying the at least one known feature (118) on a measurement device (110) in a manner that the at least one known feature (118) is reflected by the eye (302) of the person (300); wherein the at least one image (120) of the at least one reflected feature (122) in the eye (302) of the person (300) is recorded by using the measurement device (110);
    - at least one position of the at least one eye (302) of the person (300) relative to the measurement device (110); wherein the at least one position of the at least one eye (302) of the person (300) is determined by using the measurement device (110);

    b) generating outcome data (214) configured to comprise

    - at least one astigmatism parameter of the eye (302) of the person (300); wherein the at least one astigmatism parameter of the eye (302) of the person (300) is determined by comparing the at least one known feature (118) to the at least one reflected feature (122) comprised in the at least one recorded image (120) of the at least one reflected feature (122) in the eye (302) of the person (300),

    wherein the position of the at least one eye (302) of the person (300) is considered when the at least one astigmatism parameter of the eye (302) of the person (300) is determined,
    wherein the person (300) is guided to take a predetermined position of the at least one eye (302) of the person (300) relative to the measurement device (110), wherein determining the at least one astigmatism parameter comprises correcting a first deviation between the
    position of the at least one eye (302) of the person (300) and the predetermined position, wherein the at least one known feature (118) comprises a plurality of identifiable markings (126), wherein at least three different radii of the at least one eye (302) of the person (300) are determined by considering the plurality of identifiable markings (126),
    **characterised in that**
    the at least three different radii of the eye (302) of the person (300) are determined by considering at least three different distances (128) between the plurality of identifiable markings (126) in the reflected feature (122).

2.  The method according to the preceding claim, wherein determining the at least one position of the eye (302) of the person (300), in step a), comprises determining a distance (218) of the at least one eye (302) of the person (300) to the measurement device (110), and wherein determining the at least one position of the eye (302) of the person (300), in step a), comprises determining a position of the eye (302) of the person (300) in a plane (220), wherein the plane (220) is perpendicular to the distance (218).

3.  The method according to any one of the preceding claims, wherein the predetermined position is defined in a plane (220) by a paraxial model, wherein the paraxial model is defined by an isosceles triangle (222) having at least two sides of equal length, wherein a first side (224) of equal length begins at the image capturing unit (112) and ends at the at least one eye (302) of the person (300), wherein a second side (226) of equal length begins at the at least one eye (302) of the person (300) and ends at the at least one known feature (118) being displayed on at least one screen (114), wherein the predetermined position is further defined by a predetermined distance (218).

4.  The method according to any one of the preceding claims, wherein an orientation of the at least one eye (302) of the person (300) is aligned to a predetermined orientation of the at least one eye (302), wherein in the predetermined orientation of the at least one eye (302) the at least one known feature (118) is reflected on the center of the at least one eye (302) of the person (300), wherein at least one visual stimulus (124) is displayed on the measurement device (110), wherein the at least one visual stimulus (124) is configured for aligning the orientation of the at least one eye (302) of the person (300) to the predetermined orientation by causing the person (300) to direct a line of sight (304) of the at least one eye (302) of the person (300) onto the at least one visual stimulus (124).

5. The method according to any one of the preceding claims, wherein the person is guided to take the predetermined position of the at least one eye (302) of the person (300) in relation to the measurement device (110) by at least one of:

   - an audible signal;
   - a visual signal (124), wherein the visual signal (124) differs from the at least one known feature (118);
   - a tactile signal.

6. The method according to the preceding claim, wherein the visual signal (124) is displayed on the measurement device (110).

7. The method according to the preceding claim, wherein the at least one eye (302) of the person (300) is further displayed on a screen (114), wherein the person (300) is indicated to take the predetermined position in a manner that the displayed at least one eye (302) of the person (300) is to be positioned at a location indicated by the displayed visual signal (124).

8. The method according to any one of the preceding claims, wherein the at least one position of the at least one eye (302) of the person (300) is continuously determined when the person (300) is guided to take the predetermined position.

9. A computer program comprising instructions which, when the program is executed by a computer, cause the computer to carry out a computer-implemented method (200) for determining at least one astigmatism parameter of at least one eye (302) of a person (300), the method comprising the following steps:

   a) generating input data (202) configured to comprise

   - at least one known feature (118) configured for determining the at least one astigmatism parameter of the at least one eye (302) of the person (300);
   - at least one image (120) of at least one reflected feature (122) in the eye (302) of the person (300), wherein the at least one image (120) of the at least one reflected feature (122) is generated by displaying the at least one known feature (118) on a measurement device (110) in a manner that the at least one known feature (118) is reflected by the eye (302) of the person (300); wherein the at least one image (120) of the at least one reflected feature (122) in the eye (302) of the person (300) is recorded by using the measurement device (110);
   - at least one position of the at least one eye (302) of the person (300) relative to the measurement device (110); wherein the at least one position of the at least one eye (302) of the person (300) is determined by using the measurement device (110);

   b) generating outcome data (214) configured to comprise

   - at least one astigmatism parameter of the eye (302) of the person (300); wherein the at least one astigmatism parameter of the eye (302) of the person (300) is determined by comparing the at least one known feature (118) to the at least one reflected feature (122) comprised in the at least one recorded image (120) of the at least one reflected feature (122) in the eye (302) of the person (300),

   wherein the position of the at least one eye (302) of the person (300) is considered when the at least one astigmatism parameter of the eye (302) of the person (300) is determined, the person (300) is guided to take a predetermined position of the at least one eye (302) of the person (300) relative to the measurement device (110), wherein determining the at least one astigmatism parameter comprises correcting a first deviation between the position of the at least one eye (302) of the person (300) and the predetermined position, wherein the at least one known feature (118) comprises a plurality of identifiable markings (126), wherein at least three different radii of the at least one eye (302) of the person (300) are determined by considering the plurality of identifiable markings (126),
   **characterised in that**
   the at least three different radii of the eye (302) of the person (300) are determined by considering at least three different distances (128) between the plurality of identifiable markings (126) in the reflected feature (122).

10. A remote device (402) configured to generate outcome data (214) for determining at least one astigmatism parameter of at least one eye (302) of a person (300), wherein the remote device (402) is configured to receive input data provided by a field device (400) configured to generate input data (202) for determining at least one astigmatism parameter of at least one eye (302) of a person (300) by using a connection interface (404), wherein the field device (400) is configured to carry out a computer-implemented method (200) for determining at least one astigmatism parameter of at least one eye (302) of a person (300), wherein the field device (400) is used as a measurement device (110), the computer-implemented method (200) comprising the following steps:

a) generating input data (202) configured to comprise

- at least one known feature (118) configured for determining the at least one astigmatism parameter of the at least one eye (302) of the person (300);
- at least one image (120) of at least one reflected feature (122) in the eye (302) of the person (300), wherein the at least one image (120) of the at least one reflected feature (122) is generated by displaying the at least one known feature (118) on the measurement device (110) in a manner that the at least one known feature (118) is reflected by the eye (302) of the person (300); wherein the at least one image (120) of the at least one reflected feature (122) in the eye (302) of the person (300) is recorded by using the measurement device (110);
- at least one position of the at least one eye (302) of the person (300) relative to the measurement device (110); wherein the at least one position of the at least one eye (302) of the person (300) is determined by using the measurement device (110);

wherein the input data is forwarded to the remote device (402) configured for generating outcome data (214) by considering the position of the at least one eye (302) of the person (300) by using a connection interface (404), wherein the remote device (402) is configured to carry out a computer-implemented method (200) for determining at least one astigmatism parameter of at least one eye (302) of a person (300), wherein the method is comprising the following step:
b) generating outcome data (214) by using a processing device configured to comprise

- at least one astigmatism parameter of the eye (302) of the person (300); wherein the at least one astigmatism parameter of the eye (302) of the person (300) is determined by comparing at least one known feature (118) comprised by the input data (202) to at least one reflected feature (122) comprised in at least one recorded image (120) of the at least one reflected feature (122) in the eye (302) of the person (300) comprised by the by input data (202),

a position of the at least one eye (302) of the person (300) comprised by the by input data (202) is considered when the at least one astigmatism parameter of the eye (302) of the person (300) is determined, wherein

the field device (400) is configured for guiding the person (300) to take a predetermined position of the at least one eye (302) of the person (300) relative to the measurement device (110), wherein determining the at least one astigmatism parameter comprises correcting a first deviation between the position of the at least one eye (302) of the person (300) and the predetermined position, wherein the at least one known feature (118) comprises a plurality of identifiable markings (126), wherein at least three different radii of the at least one eye (302) of the person (300) are determined by considering the plurality of identifiable markings (126), **characterised in that** the at least three different radii of the eye (302) of the person (300) are determined by considering at least three different distances (128) between the plurality of identifiable markings (126) in the reflected feature (122).

11. A determining device (100) configured to determine at least one astigmatism parameter of an eye (302) of a person (300), wherein the device is configured to carry out a computer-implemented method (200) for determining at least one astigmatism parameter of an eye (302) of a person (300), wherein the determining device (100) is used as a measurement device (110), the computer-implemented method (200) comprising the following steps:

a) generating input data (202) configured to comprise

- at least one known feature (118) configured for determining the at least one astigmatism parameter of the at least one eye (302) of the person (300);
- at least one image (120) of at least one reflected feature (122) in the eye (302) of the person (300), wherein the at least one image (120) of the at least one reflected feature (122) is generated by displaying the at least one known feature (118) on the measurement device (110) in a manner that the at least one known feature (118) is reflected by the eye (302) of the person (300); wherein the at least one image (120) of the at least one reflected feature (122) in the eye (302) of the person (300) is recorded by using the measurement device (110);
- at least one position of the at least one eye (302) of the person (300) relative to the measurement device (110); wherein the at least one position of the at least one eye (302) of the person (300) is determined by using the measurement device (110);

b) generating outcome data (214) by using a processing device configured to comprise

- at least one astigmatism parameter of the eye (302) of the person (300); wherein the at least one astigmatism parameter of the eye (302) of the person (300) is determined by comparing the at least one known feature (118) to the at least one reflected feature (122) comprised in the at least one recorded image (120) of the at least one reflected feature (122) in the eye (302) of the person (300);

wherein the position of the at least one eye (302) of the person (300) is considered when the at least one astigmatism parameter of the eye (302) of the person (300) is determined,
wherein the person (300) is guided to take a predetermined position of the at least one eye (302) of the person (300) relative to the measurement device (110), wherein determining the at least one astigmatism parameter comprises correcting a first deviation between the position of the at least one eye (302) of the person (300) and the predetermined position, wherein the at least one known feature (118) comprises a plurality of identifiable markings (126), wherein at least three different radii of the at least one eye (302) of the person (300) are determined by considering the plurality of identifiable markings (126),
**characterised in that**
the at least three different radii of the eye (302) of the person (300) are determined by considering at least three different distances (128) between the plurality of identifiable markings (126) in the reflected feature (122).

12. A method for producing a geometrical model (500) of at least one spectacle lens for manufacturing of the at least one spectacle lens, wherein producing the geometrical model comprises

- producing a geometrical model of at least one spectacle lens for at least one eye (302) of a person (300) by using data related to at least one refractive value; and
- determining the data related to the at least one refractive value by carrying out a computer-implemented method (200) for determining at least one astigmatism parameter of at least one eye (302) of a person (300), the computer-implemented method (200) comprising the following steps:

a) generating input data (202) configured to comprise

- at least one known feature (118) configured for determining the at least one astigmatism parameter of the at least one eye (302) of the person (300);
- at least one image (120) of at least one reflected feature (122) in the eye (302) of the person (300), wherein the at least one image (120) of the at least one reflected feature (122) is generated by displaying the at least one known feature (118) on a measurement device (110) in a manner that the at least one known feature (118) is reflected by the eye (302) of the person (300); wherein the at least one image (120) of the at least one reflected feature (122) in the eye (302) of the person (300) is recorded by using the measurement device (110);
- at least one position of the at least one eye (302) of the person (300) relative to the measurement device (110); wherein the at least one position of the at least one eye (302) of the person (300) is determined by using the measurement device (110);

b) generating outcome data (214) by using a processing device configured to comprise

- at least one astigmatism parameter of the eye (302) of the person (300); wherein the at least one astigmatism parameter of the eye (302) of the person (300) is determined by comparing the at least one known feature (118) to the at least one reflected feature (122) comprised in the at least one recorded image (120) of the at least one reflected feature (122) in the eye (302) of the person (300);

wherein the position of the at least one eye (302) of the person (300) is considered when the at least one astigmatism parameter of the eye (302) of the person (300) is determined,
the person (300) is guided to take a predetermined position of the at least one eye (302) of the person (300) relative to the measurement device (110), wherein determining the at least one astigmatism parameter comprises correcting a first deviation between the position of the at least one eye (302) of the person (300) and the predetermined position, wherein the at least one known feature (118) comprises a plurality of identifiable markings (126), wherein at least three different radii of the at least one eye (302) of the person (300) are determined by considering the plurality of identifiable markings (126),
**characterised in that**
the at least three different radii of the eye (302) of the person (300) are determined by considering at least three different distances (128) between the plurality of identifiable markings (126) in the reflected feature (122).

13. A method for producing at least one spectacle lens (502), wherein the at least one spectacle lens is manufactured by processing at least one lens blank by using a geometrical model of the at least one spectacle lens, wherein the method comprises that the geometrical model is produced by performing the method for producing a geometrical model (500) of at least one spectacle lens according to the preceding claim.

14. A use of an external lens (130) for performing the method according to any one of the preceding method claims 1-8, 12, 13, wherein the external lens (130) is arranged on the measurement device (110).

**Patentansprüche**

1. Computerimplementiertes Verfahren (200) zum Bestimmen wenigstens eines Astigmatismusparameters wenigstens eines Auges (302) einer Person (300), wobei das Verfahren die folgenden Schritte umfasst:

   a) Erzeugen von Eingabedaten (202), die dazu konfiguriert sind, Folgendes zu umfassen:

   - wenigstens ein bekanntes Merkmal (118), das zum Bestimmen des wenigstens einen Astigmatismusparameters des wenigstens einen Auges (302) der Person (300) konfiguriert ist;
   - wenigstens ein Bild (120) wenigstens eines reflektierten Merkmals (122) in dem Auge (302) der Person (300), wobei das wenigstens eine Bild (120) des wenigstens einen reflektierten Merkmals (122) durch Anzeigen des wenigstens einen bekannten Merkmals (118) auf einer Messungsvorrichtung (110) auf eine solche Weise erzeugt wird, dass das wenigstens eine bekannte Merkmal (118) durch das Auge (302) der Person (300) reflektiert wird; wobei das wenigstens eine Bild (120) des wenigstens einen reflektierten Merkmals (122) in dem Auge (302) der Person (300) durch Verwenden der Messungsvorrichtung (110) aufgezeichnet wird;
   - wenigstens eine Position des wenigstens einen Auges (302) der Person (300) relativ zu der Messungsvorrichtung (110); wobei die wenigstens eine Position des wenigstens einen Auges (302) der Person (300) durch Verwenden der Messungsvorrichtung (110) bestimmt wird;

   b) Erzeugen von Ausgabedaten (214), die dazu konfiguriert sind, Folgendes zu umfassen:

   - wenigstens einen Astigmatismusparameter des Auges (302) der Person (300); wobei der wenigstens eine Astigmatismusparameter des Auges (302) der Person (300) durch Vergleichen des wenigstens einen bekannten Merkmals (118) mit dem wenigstens einen reflektierten Merkmal (122) bestimmt wird, das in dem wenigstens einen aufgezeichneten Bild (120) des wenigstens einen reflektierten Merkmals (122) in dem Auge (302) der Person (300) enthalten ist,

     wobei die Position des wenigstens einen Auges (302) der Person (300) betrachtet wird, wenn der wenigstens eine Astigmatismusparameter des Auges (302) der Person (300) bestimmt wird, wobei die Person (300) zum Einnehmen einer vorbestimmten Position des wenigstens eine Auges (302) der Person (300) relativ zu der Messungsvorrichtung (110) geführt wird, wobei das Bestimmen des wenigstens einen Astigmatismusparameters Korrigieren einer ersten Abweichung zwischen der Position des wenigstens einen Auges (302) der Person (300) und der vorbestimmten Position umfasst, wobei das wenigstens eine bekannte Merkmal (118) mehrere identifizierbare Markierungen (126) umfasst, wobei wenigstens drei unterschiedliche Radien des wenigstens einen Auges (302) der Person (300) bestimmt werden, indem die mehreren identifizierbaren Markierungen (126) betrachtet werden, **dadurch gekennzeichnet, dass**

die wenigstens drei unterschiedlichen Radien des Auges (302) der Person (300) bestimmt werden, indem wenigstens drei unterschiedliche Entfernungen (128) zwischen den mehreren identifizierbaren Markierungen (126) in dem reflektierten Merkmal (122) betrachtet werden.

2. Verfahren nach dem vorhergehenden Anspruch, wobei das Bestimmen der wenigstens einen Position des Auges (302) der Person (300) in Schritt a) Bestimmen einer Entfernung (218) des wenigstens einen Auges (302) der Person (300) zu der Messungsvorrichtung (110) umfasst und wobei das Bestimmen der wenigstens einen Position des Auges (302) der Person (300) in Schritt a) Bestimmen einer Position des Auges (302) der Person (300) in einer Ebene (220) umfasst, wobei die Ebene (220) senkrecht zu der Entfernung (218) ist.

3. Verfahren nach einem der vorhergehenden Ansprüche, wobei die vorbestimmte Position in einer Ebene (220) durch ein paraxiales Modell definiert wird, wobei das paraxiale Modell durch ein gleichschenkliges Dreieck (222) mit wenigstens zwei Seiten gleicher Länge definiert wird, wobei eine erste Seite (224) gleicher Länge bei der Bilderfassungseinheit (112) beginnt und bei dem wenigstens einen Auge (302) der Person (300) endet, wobei eine zweite Seite (226) gleicher Länge bei dem wenigstens einen Auge (302) der Person (300) beginnt und bei dem wenigstens einen bekannten Merkmal (118) endet, das auf wenigstens einem Bildschirm (114) angezeigt wird, wobei die vorbestimmte Position ferner durch eine vorbestimmte Entfernung (218) definiert wird.

4. Verfahren nach einem der vorhergehenden Ansprüche, wobei eine Orientierung des wenigstens einen Auges (302) der Person (300) mit einer vorbestimmten Orientierung des wenigstens einen Auges (302) ausgerichtet ist, wobei in der vorbestimmten Orientierung des wenigstens einen Auges (302) das wenigstens eine bekannte Merkmal (118) an dem Zentrum des wenigstens einen Auges (302) der Person (300) reflektiert wird, wobei wenigstens ein visueller Stimulus (124) auf der Messungsvorrichtung (110) angezeigt wird, wobei der wenigstens eine visuelle Stimulus (124) zum Ausrichten der Orientierung des wenigstens einen Auges (302) der Person (300) mit der vorbestimmten Orientierung durch Bewirken davon konfiguriert ist, dass die Person (300) eine Sichtlinie (304) des wenigstens einen Auges (302) der Person (300) auf den wenigstens einen visuellen Stimulus (124) richtet.

5. Verfahren nach einem der vorhergehenden Ansprüche, wobei die Person zum Einnehmen der vorbestimmten Position des wenigstens einen Auges (302) der Person (300) in Bezug auf die Messungsvorrichtung (110) durch wenigstens eines von Folgendem geführt wird:

- ein hörbares Signal;
- ein visuelles Signal (124), wobei sich das visuelle Signal (124) von dem wenigstens einen bekannten Merkmal (118) unterscheidet;
- ein taktiles Signal.

6. Verfahren nach dem vorhergehenden Anspruch, wobei das visuelle Signal (124) auf der Messungsvorrichtung (110) angezeigt wird.

7. Verfahren nach dem vorhergehenden Anspruch, wobei das wenigstens eine Auge (302) der Person (300) ferner auf einem Bildschirm (114) angezeigt wird, wobei der Person (300) angezeigt wird, die vorbestimmte Position auf eine solche Weise einzunehmen, dass das angezeigte wenigstens eine Auge (302) der Person (300) an einer Stelle positioniert wird, die durch das angezeigte visuelle Signal (124) angegeben wird.

8. Verfahren nach einem der vorhergehenden Ansprüche, wobei die wenigstens eine Position des wenigstens einen Auges (302) der Person (300) kontinuierlich bestimmt wird, wenn die Person (300) zum Einnehmen der vorbestimmten Position geführt wird.

9. Computerprogramm, das Anweisungen umfasst, die, wenn das Programm durch einen Computer ausgeführt wird, den Computer zum Ausführen eines computerimplementierten Verfahrens (200) zum Bestimmen wenigstens eines Astigmatismusparameters wenigstens eines Auges (302) einer Person (300) veranlassen, wobei das Verfahren die folgenden Schritte umfasst:

a) Erzeugen von Eingabedaten (202), die dazu konfiguriert sind, Folgendes zu umfassen:

- wenigstens ein bekanntes Merkmal (118), das zum Bestimmen des wenigstens einen Astigmatismusparameters des wenigstens einen Auges (302) der Person (300) konfiguriert ist;
- wenigstens ein Bild (120) wenigstens eines reflektierten Merkmals (122) in dem Auge (302) der Person (300), wobei das wenigstens eine Bild (120) des wenigstens einen reflektierten Merkmals (122) durch Anzeigen des wenigstens einen bekannten Merkmals (118) auf einer Messungsvorrichtung (110) auf eine solche Weise erzeugt wird, dass das wenigstens eine bekannte Merkmal (118) durch das Auge (302) der Person (300) reflektiert wird; wobei das wenigstens eine Bild (120) des wenigstens einen reflektierten Merkmals (122) in dem Auge (302) der Person (300) durch Verwenden der Messungsvorrichtung (110) aufgezeichnet wird;
- wenigstens eine Position des wenigstens einen Auges (302) der Person (300) relativ zu der Messungsvorrichtung (110); wobei die wenigstens eine Position des wenigstens einen Auges (302) der Person (300) durch Verwenden der Messungsvorrichtung (110) bestimmt wird;

b) Erzeugen von Ausgabedaten (214), die dazu konfiguriert sind, Folgendes zu umfassen:

- wenigstens einen Astigmatismusparameter des Auges (302) der Person (300); wobei der wenigstens eine Astigmatismusparameter des Auges (302) der Person (300) durch Vergleichen des wenigstens einen bekannten Merkmals (118) mit dem wenigstens einen reflektierten Merkmal (122) bestimmt wird, das in dem wenigstens einen aufgezeichneten Bild (120) des wenigstens einen reflektierten Merkmals (122) in dem Auge (302) der Person (300) enthalten ist,

wobei die Position des wenigstens einen Auges (302) der Person (300) betrachtet wird, wenn der wenigstens eine Astigmatismusparameter des Auges (302) der Person (300) bestimmt wird, die Person (300) zum Einnehmen einer vorbestimmten Position des wenigstens eine Auges (302) der Person (300) relativ zu der Messungsvorrichtung (110) geführt wird, wobei das Bestimmen des wenigstens einen Astigmatismusparameters Korrigieren einer ersten Abweichung zwischen der Position des wenigstens einen Auges (302) der Person (300) und der vorbestimmten Position umfasst, wobei das wenigstens eine bekannte Merkmal (118) mehrere identifizierbare Markierungen (126) umfasst, wobei wenigstens drei unterschiedliche Radien des wenigstens einen Auges (302) der Person (300) bestimmt werden, indem die mehreren identifizierbaren Markierungen (126) betrachtet werden, **dadurch gekennzeichnet, dass** die wenigstens drei unterschiedlichen Radien des Auges (302) der Person (300) bestimmt werden, indem wenigstens drei unterschiedliche Entfernungen (128) zwischen den mehreren identifizierbaren Markierungen (126) in dem reflektierten Merkmal (122) betrachtet werden.

10. Remote-Vorrichtung (402), die zum Erzeugen von Ausgabedaten (214) zum Bestimmen wenigstens eines Astigmatismusparameters wenigstens eines Auges (302) einer Person (300) konfiguriert ist, wobei die Remote-Vorrichtung (402) zum Empfangen von Eingabedaten konfiguriert ist, die durch eine Feldvorrichtung (400) bereitgestellt werden, die zum Erzeugen von Eingabedaten (202) zum Bestimmen wenigstens eines Astigmatismusparameters wenigstens eines Auges (302) einer Person (300) konfiguriert ist, indem eine Verbindungsschnittstelle (404) verwendet wird, wobei die Feldvorrichtung (400) zum Ausführen eines computerimplementierten Verfahrens (200) zum Bestimmen wenigstens eines Astigmatismusparameters wenigstens eines Auges (302) einer Person (300) konfiguriert ist, wobei die Feldvorrichtung (400) als eine Messungsvorrichtung (110) verwendet wird, wobei das computerimplementierte Verfahren (200) die folgenden Schritte umfasst:

a) Erzeugen von Eingabedaten (202), die dazu konfiguriert sind, Folgendes zu umfassen:

- wenigstens ein bekanntes Merkmal (118), das zum Bestimmen des wenigstens einen Astigmatismusparameters des wenigstens einen Auges (302) der Person (300) konfiguriert ist;
- wenigstens ein Bild (120) wenigstens eines reflektierten Merkmals (122) in dem Auge (302) der Person (300), wobei das wenigstens eine Bild (120) des wenigstens einen reflektierten Merkmals (122) durch Anzeigen des wenigstens einen bekannten Merkmals (118) auf der Messungsvorrichtung (110) auf eine solche Weise erzeugt wird, dass das wenigstens eine bekannte Merkmal (118) durch das Auge (302) der Person (300) reflektiert wird; wobei das wenigstens eine Bild (120) des wenigstens einen reflektierten Merkmals (122) in dem Auge (302) der Person (300) durch Verwenden der Messungsvorrichtung (110) aufgezeichnet wird;
- wenigstens eine Position des wenigstens einen Auges (302) der Person (300) relativ zu der Messungsvorrichtung (110); wobei die wenigstens eine Position des wenigstens einen Auges (302) der Person (300) durch Verwenden der Messungsvorrichtung (110) bestimmt wird;

wobei die Eingabedaten an die Remote-Vorrichtung (402) weitergeleitet werden, die zum Erzeugen von Ausgabedaten (214) durch Betrachten der Position des wenigstens einen Auges (302) der Person (300) konfiguriert ist, indem eine Verbindungsschnittstelle (404) verwendet wird,

wobei die Remote-Vorrichtung (402) zum Ausführen eines computerimplementierten Verfahrens (200) zum Bestimmen wenigstens eines Astigmatismusparameters wenigstens eines Auges (302) einer Person (300) konfiguriert ist, wobei das Verfahren den folgenden Schritt umfasst:

b) Erzeugen, durch Verwenden einer Verarbeitungsvorrichtung, von Ausgabedaten (214), die dazu konfiguriert sind, Folgendes zu umfassen:

- wenigstens einen Astigmatismusparameter des Auges (302) der Person (300); wobei der wenigstens eine Astigmatismusparameter des Auges (302) der Person (300) durch Vergleichen wenigstens eines bekannten Merkmals (118), das in den Eingabedaten (202) enthalten ist, mit wenigstens einem reflektierten Merkmal (122) bestimmt wird, das in wenigstens einem aufgezeichneten Bild (120) des wenigstens einen reflektierten Merkmals (122) in dem Auge (302) der Person (300) enthalten ist, welches in den Eingabedaten (202) enthalten ist,

wobei eine Position des wenigstens einen Auges (302) der Person (300), das in den Eingabedaten (202) enthalten ist, betrachtet wird, wenn der wenigstens eine Astigmatismusparameter des Auges (302) der Person (300) bestimmt wird,

wobei die Feldvorrichtung (400) zum Führen der Person (300) zum Einnehmen einer vorbestimmten Position des wenigstens eine Auges (302) der Person (300) relativ zu der Messungsvorrichtung (110) konfiguriert ist, wobei das Bestimmen des wenigstens einen Astigmatismusparameters Korrigieren einer ersten Abweichung zwischen der Position des wenigstens einen Auges (302) der Person (300) und der vorbestimmten Position umfasst, wobei das wenigstens eine bekannte Merkmal (118) mehrere identifizierbare Markierungen (126) umfasst, wobei wenigstens drei unterschiedliche Radien des wenigstens einen Auges (302) der Person (300) bestimmt werden, indem die mehreren identifizierbaren Markierungen (126) betrachtet werden, **dadurch gekennzeichnet, dass** die wenigstens drei unterschiedlichen Radien des Auges (302) der Person (300) bestimmt werden, indem wenigstens drei unterschiedliche Entfernungen (128) zwischen den mehreren identifizierbaren Markierungen (126) in dem reflektierten Merkmal (122) betrachtet werden.

11. Bestimmungsvorrichtung (100), die zum Bestimmen wenigstens eins Astigmatismusparameters eines Auges (302) einer Person (300) konfiguriert ist, wobei die Vorrichtung zum Ausführen eines computerimplementierten Verfahrens (200) zum Bestimmen wenigstens eines Astigmatismusparameters eines Auges (302) einer Person (300) konfiguriert ist, wobei die Bestimmungsvorrichtung (100) als eine Messungsvorrichtung (110) verwendet wird, wobei das computerimplementierte Verfahren (200) die folgenden Schritte umfasst:

a) Erzeugen von Eingabedaten (202), die dazu konfiguriert sind, Folgendes zu umfassen:

- wenigstens ein bekanntes Merkmal (118), das zum Bestimmen des wenigstens einen Astigmatismusparameters des wenigstens einen Auges (302) der Person (300) konfiguriert ist;
- wenigstens ein Bild (120) wenigstens eines reflektierten Merkmals (122) in dem Auge (302) der Person (300), wobei das wenigstens eine Bild (120) des wenigstens einen reflektierten Merkmals (122) durch Anzeigen des wenigstens einen bekannten Merkmals (118) auf der Messungsvorrichtung (110) auf eine solche Weise erzeugt wird, dass das wenigstens eine bekannte Merkmal (118) durch das Auge (302) der Person (300) reflektiert wird; wobei das wenigstens eine Bild (120) des wenigstens einen reflektierten Merkmals (122) in dem Auge (302) der Person (300) durch Verwenden der Messungsvorrichtung (110) aufgezeichnet wird,
- wenigstens eine Position des wenigstens einen Auges (302) der Person (300) relativ zu der Messungsvorrichtung (110); wobei die wenigstens eine Position des wenigstens einen Auges (302) der Person (300) durch Verwenden der Messungsvorrichtung (110) bestimmt wird;

b) Erzeugen, durch Verwenden einer Verarbeitungsvorrichtung, von Ausgabedaten (214), die dazu konfiguriert sind, Folgendes zu umfassen:

- wenigstens einen Astigmatismusparameter des Auges (302) der Person (300); wobei der wenigstens eine Astigmatismusparameter des Auges (302) der Person (300) durch Vergleichen des wenigstens einen bekannten Merkmals (118) mit dem wenigstens einen reflektierten Merkmal (122) bestimmt wird, das in dem wenigstens einen aufgezeichneten Bild (120) des wenigstens einen reflektierten Merkmals (122) in dem Auge (302) der Person (300) enthalten ist;

wobei die Position des wenigstens einen Auges (302) der Person (300) betrachtet wird, wenn der wenigstens eine Astigmatismusparameter des Auges (302) der Person (300) bestimmt wird, wobei die Person (300) zum Einnehmen einer vorbestimmten Position des wenigstens eine Auges (302) der Person (300) relativ zu der Messungsvorrichtung (110) geführt wird, wobei das Bestimmen des wenigstens einen Astigmatismusparameters Korrigieren einer ersten Abweichung zwischen der Position des wenigstens einen Auges (302) der Person (300) und der vorbestimmten Position umfasst, wobei das wenigstens eine bekannte Merkmal (118) mehrere identifizierbare Markierungen (126) umfasst, wobei wenigstens drei unterschiedliche Radien des wenigstens einen Auges (302) der Person (300) bestimmt werden, indem die mehreren identifizierbaren Markierungen (126) betrachtet werden, **dadurch gekennzeichnet, dass** die wenigstens drei unterschiedlichen Radien des Auges (302) der Person (300) bestimmt werden, indem wenigstens drei unterschiedliche Entfernungen (128) zwischen den mehreren identifizierbaren Markierungen (126) in dem reflektierten Merkmal (122) betrachtet werden.

12. Verfahren zum Produzieren eines geometrischen Modells (500) wenigstens eines Brillenglases zum Herstellen des wenigstens einen Brillenglases, wobei das Produzieren des geometrischen Modells Folgendes umfasst:

- Produzieren eines geometrischen Modells wenigstens eines Brillenglases für wenigstens ein Auge (302) einer Person (300) durch Verwenden von Daten bezüglich wenigstens eines Brechungswertes; und
- Bestimmen der Daten bezüglich des wenigstens einen Brechungswertes durch Ausführen eines computerimplementierten Verfahrens (200) zum Bestimmen wenigstens eines Astigmatismusparameters wenigstens eines Auges (302) einer Person (300), wobei das computerimplementierte Verfahren (200) die folgenden Schritte umfasst:

a) Erzeugen von Eingabedaten (202), die dazu konfiguriert sind, Folgendes zu umfassen:

- wenigstens ein bekanntes Merkmal (118), das zum Bestimmen des wenigstens einen Astigmatismusparameters des wenigstens einen Auges (302) der Person (300) konfiguriert ist;
- wenigstens ein Bild (120) wenigstens eines reflektierten Merkmals (122) in dem Auge (302) der Person (300), wobei das wenigstens eine Bild (120) des wenigstens einen reflektierten Merkmals (122) durch Anzeigen des wenigstens einen bekannten Merkmals (118) auf einer Messungsvorrichtung (110) auf eine solche Weise erzeugt wird, dass das wenigstens eine bekannte Merkmal (118) durch das Auge (302) der Person (300) reflektiert wird; wobei das wenigstens eine Bild (120) des wenigstens einen reflektierten Merkmals (122) in dem Auge (302) der Person (300) durch Verwenden der Messungsvorrichtung (110) aufgezeichnet wird;
- wenigstens eine Position des wenigstens einen Auges (302) der Person (300) relativ zu der Messungsvorrichtung (110); wobei die wenigstens eine Position des wenigstens einen Auges (302) der Person (300) durch Verwenden der Messungsvorrichtung (110) bestimmt wird;

b) Erzeugen, durch Verwenden einer Verarbeitungsvorrichtung, von Ausgabedaten (214), die dazu konfiguriert sind, Folgendes zu umfassen:

- wenigstens einen Astigmatismusparameter des Auges (302) der Person (300); wobei der wenigstens eine Astigmatismusparameter des Auges (302) der Person (300) durch Vergleichen des wenigstens einen bekannten Merkmals (118) mit dem wenigstens einen reflektierten Merkmal (122) bestimmt wird, das in dem wenigstens einen aufgezeichneten Bild (120) des wenigstens einen reflektierten Merkmals (122) in dem Auge (302) der Person (300) enthalten ist;

wobei die Position des wenigstens einen Auges (302) der Person (300) betrachtet wird, wenn der wenigstens eine Astigmatismusparameter des Auges (302) der Person (300) bestimmt wird, die Person (300) zum Einnehmen einer vorbestimmten Position des wenigstens eine Auges (302) der Person (300) relativ zu der Messungsvorrichtung (110) geführt wird, wobei das Bestimmen des wenigstens einen Astigmatismusparameters Korrigieren einer ersten Abweichung zwischen der Position des wenigstens einen Auges (302) der Person (300) und der vorbestimmten Position umfasst, wobei das wenigstens eine bekannte Merkmal (118) mehrere identifizierbare Markierungen (126) umfasst, wobei wenigstens drei unterschiedliche Radien des wenigstens einen Auges (302) der Person (300) bestimmt werden, indem die mehreren identifizierbaren Markierungen (126) betrachtet werden, **dadurch gekennzeichnet, dass**

die wenigstens drei unterschiedlichen Radien des Auges (302) der Person (300) bestimmt werden, indem wenigstens drei unterschiedliche Entfernungen (128) zwischen den mehreren identifizierbaren Markierungen (126) in dem reflektierten Merkmal (122) betrachtet werden.

13. Verfahren zum Produzieren wenigstens eines Brillenglases (502), wobei das wenigstens eine Brillenglas durch Verarbeiten wenigstens eines Linsenrohlings durch Verwenden eines geometrischen Modells des wenigstens einen Brillenglases hergestellt wird, wobei das Verfahren umfasst, dass das geometrische Modell durch Durchführen des Verfahrens zum Produzieren eines geometrischen Modells (500) wenigstens eines Brillenglases nach dem vorhergehenden Anspruch produziert wird.

14. Verwendung einer externen Linse (130) zum Durchführen des Verfahrens nach einem der vorhergehenden Ansprüche 1-8, 12, 13, wobei die externe Linse (130) auf der Messungsvorrichtung (110) angeordnet ist.


**Revendications**

1. Procédé (200) mis en œuvre par ordinateur pour déterminer au moins un paramètre d'astigmatisme d'au moins un œil (302) d'une personne (300), le procédé comprenant les étapes suivantes :

   a) la génération de données d'entrée (202) configurées pour comprendre

   - au moins une caractéristique connue (118) configurée pour déterminer le ou les paramètres d'astigmatisme de l'au moins un œil (302) de la personne (300) ;
   - au moins une image (120) d'au moins une caractéristique réfléchie (122) dans l'œil (302) de la personne (300), dans lequel la ou les images (120) de la ou des caractéristiques réfléchies (122) sont générées en affichant la ou les caractéristiques connues (118) sur un dispositif de mesure (110) de manière que la ou les caractéristiques connues (118) soient réfléchies par l'œil (302) de la personne (300) ; dans lequel la ou les images (120) du ou des éléments réfléchis (122) dans l'œil (302) de la personne (300) sont enregistrées au moyen du dispositif de mesure (110) ;
   - au moins une position de l'au moins un œil (302) de la personne (300) par rapport au dispositif de mesure (110) ; dans lequel la ou les positions de l'au moins un œil (302) de la personne (300) sont déterminées à l'aide du dispositif de mesure (110) ;

   b) la génération de données de résultat (214) configurées pour comprendre

   - au moins un paramètre d'astigmatisme de l'œil (302) de la personne (300) ; dans lequel le ou les paramètres d'astigmatisme de l'œil (302) de la personne (300) sont déterminés en comparant la ou les caractéristiques connues (118) à la ou aux caractéristiques réfléchies (122) comprises dans la ou les images (120) enregistrées de la ou des caractéristiques réfléchies (122) dans l'œil (302) de la personne (300),

   dans lequel la position de l'au moins un œil (302) de la personne (300) est prise en compte lorsque le ou les paramètres d'astigmatisme de l'œil (302) de la personne (300) sont déterminés, dans lequel la personne (300) est guidée pour prendre une position prédéterminée de l'au moins un œil (302) de la personne (300) par rapport au dispositif de mesure (110), dans lequel la détermination du ou des paramètres d'astigmatisme comprend la correction d'un premier écart entre la position de l'au moins un œil (302) de la personne (300) et la position prédéterminée, dans lequel la ou les caractéristiques connues (118) comprennent une pluralité de marquages identifiables (126), dans lequel au moins trois rayons différents de l'au moins un œil (302) de la personne (300) sont déterminés en prenant en compte la pluralité de marquages identifiables (126), **caractérisé en ce que** les au moins trois rayons différents de l'œil (302) de la personne (300) sont déterminés en prenant en compte au moins trois distances différentes (128) entre la pluralité de marquages identifiables (126) dans la caractéristique réfléchie (122).

2. Procédé selon la revendication précédente, dans lequel la détermination de la ou des positions de l'œil (302) de la personne (300), à l'étape a), comprend la détermination d'une distance (218) de l'au moins un œil (302) de la personne (300) au dispositif de mesure (110), et dans lequel la détermination de la ou des positions de l'œil (302) de la personne (300), à l'étape a), comprend la détermination d'une position de l'œil (302) de la personne (300) dans un plan (220), dans lequel le plan (220) est perpendiculaire à la distance (218).

3. Procédé selon l'une quelconque des revendications précédentes, dans lequel la position prédéterminée est définie dans un plan (220) par un modèle paraxial, dans lequel le modèle paraxial est défini par un triangle isocèle (222) ayant au moins deux côtés de longueur égale, dans lequel un premier côté (224) de longueur égale commence au niveau de l'unité de capture d'image (112) et se termine au niveau de l'au moins un œil (302) de la personne (300), dans lequel un deuxième côté (226) de même longueur commence au niveau de l'au moins un œil (302) de la personne (300) et se termine au niveau de la ou des caractéristiques connues (118) affichées sur au moins un écran (114), la position prédéterminée étant en outre définie par une distance prédéterminée (218).

4. Procédé selon l'une quelconque des revendications précédentes, dans lequel une orientation de l'au moins un œil (302) de la personne (300) est alignée sur une orientation prédéterminée de l'au moins un œil (302), dans lequel, dans l'orientation prédéterminée de l'au moins un œil (302), la ou les caractéristiques connues (118) sont réfléchies sur le centre de l'au moins un œil (302) de la personne (300), dans lequel au moins un stimulus visuel (124) est affiché sur le dispositif de mesure (110), dans lequel le ou les stimuli visuels (124) sont configurés pour aligner l'orientation de l'au moins un œil (302) de la personne (300) sur l'orientation prédéterminée en amenant la personne (300) à diriger une ligne de visée (304) de l'au moins un œil (302) de la personne (300) sur le ou les stimuli visuels (124).

5. Procédé selon l'une quelconque des revendications précédentes, dans lequel la personne est guidée pour prendre la position prédéterminée de l'au moins un œil (302) de la personne (300) par rapport au dispositif de mesure (110) par au moins l'un parmi :

   - un signal sonore ;
   - un signal visuel (124), dans lequel le signal visuel (124) diffère de la ou des caractéristiques connues (118) ;
   - un signal tactile.

6. Procédé selon la revendication précédente, dans lequel le signal visuel (124) est affiché sur le dispositif de mesure (110).

7. Procédé selon la revendication précédente, dans lequel l'au moins un œil (302) de la personne (300) est en outre affiché sur un écran (114), dans lequel la personne (300) est invitée à prendre la position prédéterminée d'une manière telle que l'au moins un œil (302) affiché de la personne (300) soit positionné à un emplacement indiqué par le signal visuel affiché (124).

8. Procédé selon l'une quelconque des revendications précédentes, dans lequel la ou les positions de l'au moins un œil (302) de la personne (300) sont déterminées en continu lorsque la personne (300) est guidée pour prendre la position prédéterminée.

9. Programme d'ordinateur comprenant des instructions qui, lorsque le programme est exécuté par un ordinateur, amènent l'ordinateur à exécuter un procédé (200) mis en œuvre par ordinateur pour déterminer au moins un paramètre d'astigmatisme d'au moins un œil (302) d'une personne (300), le procédé comprenant les étapes suivantes :

   a) la génération de données d'entrée (202) configurées pour comprendre

      - au moins une caractéristique connue (118) configurée pour déterminer le ou les paramètres d'astigmatisme de l'au moins un œil (302) de la personne (300) ;
      - au moins une image (120) d'au moins une caractéristique réfléchie (122) dans l'œil (302) de la personne (300), dans lequel la ou les images (120) de la ou des caractéristiques réfléchies (122) sont générées en affichant la ou les caractéristiques connues (118) sur un dispositif de mesure (110) de manière que la ou les caractéristiques connues (118) soient réfléchies par l'œil (302) de la personne (300) ; dans lequel la ou les images (120) du ou des éléments réfléchis (122) dans l'œil (302) de la personne (300) sont enregistrées au moyen du dispositif de mesure (110) ;
      - au moins une position de l'au moins un œil (302) de la personne (300) par rapport au dispositif de mesure (110) ; dans lequel la ou les positions de l'au moins un œil (302) de la personne (300) sont déterminées à l'aide du dispositif de mesure (110) ;

b) la génération de données de résultat (214) configurées pour comprendre

- au moins un paramètre d'astigmatisme de l'œil (302) de la personne (300) ; dans lequel le ou les paramètres d'astigmatisme de l'œil (302) de la personne (300) sont déterminés en comparant la ou les caractéristiques connues (118) à la ou aux caractéristiques réfléchies (122) comprises dans la ou les images (120) enregistrées de la ou des caractéristiques réfléchies (122) dans l'œil (302) de la personne (300),

dans lequel la position de l'au moins un œil (302) de la personne (300) est prise en compte lorsque le ou les paramètres d'astigmatisme de l'œil (302) de la personne (300) sont déterminés, la personne (300) est guidée pour prendre une position prédéterminée de l'au moins un œil (302) de la personne (300) par rapport au dispositif de mesure (110), dans lequel la détermination du ou des paramètres d'astigmatisme comprend la correction d'un premier écart entre la position de l'au moins un œil (302) de la personne (300) et la position prédéterminée, dans lequel la ou les caractéristiques connues (118) comprennent une pluralité de marquages identifiables (126), dans lequel au moins trois rayons différents de l'au moins un œil (302) de la personne (300) sont déterminés en prenant en compte la pluralité de marquages identifiables (126), **caractérisé en ce que** les au moins trois rayons différents de l'œil (302) de la personne (300) sont déterminés en prenant en compte au moins trois distances différentes (128) entre la pluralité de marquages identifiables (126) dans la caractéristique réfléchie (122).

10. Dispositif distant (402) configuré pour générer des données de résultat (214) pour déterminer au moins un paramètre d'astigmatisme d'au moins un œil (302) d'une personne (300), dans lequel le dispositif distant (402) est configuré pour recevoir des données d'entrée fournies par un dispositif sur site (400) configuré pour générer des données d'entrée (202) pour déterminer au moins un paramètre d'astigmatisme d'au moins un œil (302) d'une personne (300) à l'aide d'une interface de connexion (404), dans lequel le dispositif sur site (400) est configuré pour exécuter un procédé mis en œuvre par ordinateur (200) pour déterminer au moins un paramètre d'astigmatisme d'au moins un œil (302) d'une personne (300), dans lequel le dispositif sur site (400) est utilisé comme dispositif de mesure (110), le procédé (200) mis en œuvre par ordinateur comprenant les étapes suivantes :

a) la génération de données d'entrée (202) configurées pour comprendre

- au moins une caractéristique connue (118) configurée pour déterminer le ou les paramètres d'astigmatisme de l'au moins un œil (302) de la personne (300) ;
- au moins une image (120) d'au moins une caractéristique réfléchie (122) dans l'œil (302) de la personne (300), dans lequel la ou les images (120) de la ou des caractéristiques réfléchies (122) sont générées en affichant la ou les caractéristiques connues (118) sur le dispositif de mesure (110) de manière que la ou les caractéristiques connues (118) soient réfléchies par l'œil (302) de la personne (300) ; dans lequel la ou les images (120) du ou des éléments réfléchis (122) dans l'œil (302) de la personne (300) sont enregistrées au moyen du dispositif de mesure (110) ;
- au moins une position de l'au moins un œil (302) de la personne (300) par rapport au dispositif de mesure (110) ; dans lequel la ou les positions de l'au moins un œil (302) de la personne (300) sont déterminées à l'aide du dispositif de mesure (110) ;

dans lequel les données d'entrée sont transférées au dispositif distant (402) configuré pour générer des données de résultat (214) en prenant en compte la position de l'au moins un œil (302) de la personne (300) à l'aide d'une interface de connexion (404), dans lequel le dispositif (402) distant est configuré pour exécuter un procédé (200) mis en œuvre par ordinateur pour déterminer au moins un paramètre d'astigmatisme d'au moins un œil (302) d'une personne (300), dans lequel le procédé comprend l'étape suivante :

b) la génération de données de résultat (214) à l'aide d'un dispositif de traitement configuré pour comprendre

- au moins un paramètre d'astigmatisme de l'œil (302) de la personne (300) ; dans lequel le ou les paramètres d'astigmatisme de l'œil (302) de la personne (300) sont déterminés en comparant au moins une caractéristique connue (118) comprise dans les données d'entrée (202) à au moins une caractéristique réfléchie (122) comprise dans au moins une image enregistrée (120) de la ou des caractéristiques réfléchies (122) dans l'œil (302) de la personne (300) comprises dans les données d'entrée (202), une position de l'au moins un œil (302) de la personne (300) comprises dans les données d'entrée (202) est prise en compte lorsque le ou les paramètres d'astigmatisme de l'œil (302) de la personne (300) sont déterminés,

dans lequel le dispositif sur site (400) est configuré pour guider la personne (300) pour prendre une position prédéterminée de l'au moins un œil (302) de la personne (300) par rapport au dispositif de mesure (110), dans lequel la détermination du ou des paramètres d'astigmatisme comprend la correction d'un premier écart entre la position de l'au moins un œil (302) de la personne (300) et la position prédéterminée, dans lequel la ou les caractéristiques connues (118) comprennent une pluralité de marquages identifiables (126), dans lequel au moins trois rayons différents de l'au moins un œil (302) de la personne (300) sont déterminés en prenant en compte la pluralité de marquages identifiables (126), **caractérisé en ce que** les au moins trois rayons différents de l'œil (302) de la personne (300) sont déterminés en prenant en compte au moins trois distances différentes (128) entre la pluralité de marquages identifiables (126) dans la caractéristique réfléchie (122).

11. Dispositif de détermination (100) configuré pour déterminer au moins un paramètre d'astigmatisme d'un œil (302) d'une personne (300), dans lequel le dispositif est configuré pour exécuter un procédé mis en œuvre par ordinateur (200) de détermination d'au moins un paramètre d'astigmatisme d'un œil (302) d'une personne (300), dans lequel le dispositif de détermination (100) est utilisé comme dispositif de mesure (110), le procédé mis en œuvre par ordinateur (200) comprenant les étapes suivantes :

a) la génération de données d'entrée (202) configurées pour comprendre

- au moins une caractéristique connue (118) configurée pour déterminer le ou les paramètres d'astigmatisme de l'au moins un œil (302) de la personne (300) ;
- au moins une image (120) d'au moins une caractéristique réfléchie (122) dans l'œil (302) de la personne (300), dans lequel la ou les images (120) de la ou des caractéristiques réfléchies (122) sont générées en affichant la ou les caractéristiques connues (118) sur le dispositif de mesure (110) de manière que la ou les caractéristiques connues (118) soient réfléchies par l'œil (302) de la personne (300) ; dans lequel la ou les images (120) du ou des éléments réfléchis (122) dans l'œil (302) de la personne (300) sont enregistrées au moyen du dispositif de mesure (110) ;
- au moins une position de l'au moins un œil (302) de la personne (300) par rapport au dispositif de mesure (110) ; dans lequel la ou les positions de l'au moins un œil (302) de la personne (300) sont déterminées à l'aide du dispositif de mesure (110) ;

b) la génération de données de résultat (214) à l'aide d'un dispositif de traitement configuré pour comprendre

- au moins un paramètre d'astigmatisme de l'œil (302) de la personne (300) ; dans lequel le ou les paramètres d'astigmatisme de l'œil (302) de la personne (300) sont déterminés en comparant la ou les caractéristiques connues (118) à la ou aux caractéristiques réfléchies (122) comprises dans la ou les images (120) enregistrées de la ou des caractéristiques réfléchies (122) dans l'œil (302) de la personne (300) ;

dans lequel la position de l'au moins un œil (302) de la personne (300) est prise en compte lorsque le ou les paramètres d'astigmatisme de l'œil (302) de la personne (300) sont déterminés, dans lequel la personne (300) est guidée pour prendre une position prédéterminée de l'au moins un œil (302) de la personne (300) par rapport au dispositif de mesure (110), dans lequel la détermination du ou des paramètres d'astigmatisme comprend la correction d'un premier écart entre la position de l'au moins un œil (302) de la personne (300) et la position prédéterminée, dans lequel la ou les caractéristiques connues (118) comprennent une pluralité de marquages identifiables (126), dans lequel au moins trois rayons différents de l'au moins un œil (302) de la personne (300) sont déterminés en prenant en compte la pluralité de marquages identifiables (126), **caractérisé en ce que** les au moins trois rayons différents de l'œil (302) de la personne (300) sont déterminés en prenant en compte au moins trois distances différentes (128) entre la pluralité de marquages identifiables (126) dans la caractéristique réfléchie (122).

12. Procédé de production d'un modèle géométrique (500) d'au moins un verre de lunettes pour la fabrication d'au moins un verre de lunettes, dans lequel la production du modèle géométrique comprend

- la production d'un modèle géométrique d'au moins un verre de lunettes pour au moins un œil (302) d'une personne (300) en utilisant des données relatives à au moins une valeur de réfraction ; et
- la détermination des données relatives à la ou aux valeurs de réfraction en exécutant un procédé (200) mis en œuvre par ordinateur pour déterminer au moins un paramètre d'astigmatisme d'au moins un œil (302) d'une personne (300), le procédé (200) mis en œuvre par ordinateur comprenant les étapes suivantes :

a) la génération de données d'entrée (202) configurées pour comprendre

- au moins une caractéristique connue (118) configurée pour déterminer le ou les paramètres d'astigmatisme de l'au moins un œil (302) de la personne (300) ;
- au moins une image (120) d'au moins une caractéristique réfléchie (122) dans l'œil (302) de la personne (300), dans lequel la ou les images (120) de la ou des caractéristiques réfléchies (122) sont générées en affichant la ou les caractéristiques connues (118) sur un dispositif de mesure (110) de manière que la ou les caractéristiques connues (118) soient réfléchies par l'œil (302) de la personne (300) ; dans lequel la ou les images (120) du ou des éléments réfléchis (122) dans l'œil (302) de la personne (300) sont enregistrées au moyen du dispositif de mesure (110) ;
- au moins une position de l'au moins un œil (302) de la personne (300) par rapport au dispositif de mesure (110) ; dans lequel la ou les positions de l'au moins un œil (302) de la personne (300) sont déterminées à l'aide du dispositif de mesure (110) ;

b) la génération de données de résultat (214) à l'aide d'un dispositif de traitement configuré pour comprendre

- au moins un paramètre d'astigmatisme de l'œil (302) de la personne (300) ; dans lequel le ou les paramètres d'astigmatisme de l'œil (302) de la personne (300) sont déterminés en comparant la ou les caractéristiques connues (118) à la ou aux caractéristiques réfléchies (122) comprises dans la ou les images (120) enregistrées de la ou des caractéristiques réfléchies (122) dans l'œil (302) de la personne (300) ;

dans lequel la position de l'au moins un œil (302) de la personne (300) est prise en compte lorsque le ou les paramètres d'astigmatisme de l'œil (302) de la personne (300) sont déterminés, la personne (300) est guidée pour prendre une position prédéterminée de l'au moins un œil (302) de la personne (300) par rapport au dispositif de mesure (110), dans lequel la détermination du ou des paramètres d'astigmatisme comprend la correction d'un premier écart entre la position de l'au moins un œil (302) de la personne (300) et la position prédéterminée, dans lequel la ou les caractéristiques connues (118) comprennent une pluralité de marquages identifiables (126), dans lequel au moins trois rayons différents de l'au moins un œil (302) de la personne (300) sont déterminés en prenant en compte la pluralité de marquages identifiables (126), **caractérisé en ce que** les au moins trois rayons différents de l'œil (302) de la personne (300) sont déterminés en prenant en compte au moins trois distances différentes (128) entre la pluralité de marquages identifiables (126) dans la caractéristique réfléchie (122).

13. Procédé de fabrication d'au moins un verre de lunettes (502), dans lequel l'au moins un verre de lunettes est fabriqué en traitant au moins une ébauche de verre en utilisant un modèle géométrique du ou des verres de lunettes, le procédé comprenant le fait que le modèle géométrique est produit par l'exécution du procédé de fabrication d'un modèle géométrique (500) d'au moins un verre de lunettes selon la revendication précédente.

14. Utilisation d'un verre externe (130) pour exécuter le procédé selon l'une quelconque des revendications 1 à 8, 12, 13 de procédé précédentes, dans laquelle le verre externe (130) est disposé sur le dispositif de mesure (110).

EP 4 586 881 B1

Fig. 2

200

202

210 → 212 → 204 → 206 → 208 →

214

216

Fig. 1

100, 110

112
114
302
116

300

39

Fig. 3

Fig. 4

Fig. 5

Fig. 6

Fig. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9833140 B2
- US 20200237210 A1
- US 20170172406 A1
- US 20170164827 A1
- US 20140268060 A1
- DE 102015100147 A1
- KR 101784599 B1
- WO 2022090376 A1